Europäisches Patentamt

European Patent Office

Office européen des brevets

(11) Publication number: **0 207 751**
**B1**

(12) **EUROPEAN PATENT SPECIFICATION**

(45) Date of publication of patent specification: **14.11.90**

(21) Application number: **86304998.7**

(22) Date of filing: **27.06.86**

(51) Int. Cl.⁵: **C 07 K 15/14,** C 12 N 15/00, C 07 H 21/04, C 12 P 21/00, C 12 N 1/20, A 61 K 37/02 // (C12N1/20, C12R1:19)

(54) Fibronectins.

(30) Priority: **28.06.85 GB 8516421**

(43) Date of publication of application: **07.01.87 Bulletin 87/02**

(45) Publication of the grant of the patent: **14.11.90 Bulletin 90/46**

(84) Designated Contracting States: **AT BE CH DE FR GB IT LI LU NL SE**

(56) References cited:
EP-A-0 022 052
EP-A-0 114 685
WO-A-84/00540

ANNUAL REVIEW OF BIOCHEMISTRY, vol. 52, 1983, (Palo Alto, USA) K.M.Yamada "Cell Surface Interactions with Extracellular Materials" pages 761-799

(73) Proprietor: **Delta Biotechnology Limited**
**137 High Street**
**Burton on Trent DE14 1JZ (GB)**

(72) Inventor: **Baralle, Francisco E.**
**178 The Moors**
**Kidlington Oxford OX5 (GB)**

(74) Representative: **Bassett, Richard Simon et al**
**ERIC POTTER & CLARKSON St. Mary's Court St. Mary's Gate**
**Nottingham NG1 1LE (GB)**

(56) References cited:
PROCEEDINGS OF THE NATIONAL ACADEMY OF SCIENCES OF THE UNITED STATES OF AMERICA, vol. 80, no. 11, June 1983 (Baltimore, USA) A.R.KORNBLIHTT et al. "Isolation and characterization of cDNA clones for human and bovine fibronectins" pages 3218-3222

CELL, vol. 35, no.2, December 1983 (Part 1) (Cambridge, Mass., USA) J.E.SCHWARZBAUER et al. "Three Different Fibronectin mRNAs Arise by Alternative Splicing within the Coding Region" pages 421-431

Courier Press, Leamington Spa, England.

(56) References cited:
Petersen et al. (1983), Proc. Natl. Acad. Sci USA, 80: 137-141

Smith et al. (1982), J. Biol. Chem., 257: 6518-6523

Ruoslashti et al (1979), J. Biol. Chem., 254: 6054-6059

Bernard et al (1985), Biochemistry, 24: *2695-2704

## Description

Fibronectins (FNs) constitute a class of high molecular weight glyco-proteins that have a key role in various contact processes of the vertebrates such as cell attachment and spreading, cell migration, control of cell morphology, differentiation and oncogenic transformation. All these biological activities imply interaction of FN with cells and with extracellular materials. Binding activities for collagen, heparin, fibrin, cell surfaces, bacteria and DNA have been located in different domains of the FN molecule (for review, see Yamada, 1983).

FN is one of the most versatile known proteins, both functionally and structurally. FN molecules are usually dimers of similar but not identical polypeptides of MW≃250,000. Cellular FN is found in a fibrilar component of the extracellular matrix of fibroblasts and other cell types. Plasma FN is a soluble molecule present in high concentrations in plasma (300 μg/ml) and probably involved in opsonization, wound healing and haemostasis (Yamada, 1983; Hynes & Yamada, 1982). Partial primary structure data have revealed high conserved amino acid sequences both between the two FN forms and among FNs from different species: bovine plasma (Petersen et al, 1983), bovine cellular (Kornblihtt et al, 1983), human plasma (Pande & Shively 1982; Garcia-Pardo et al, 1983), human cellular (Kornblihtt et al, 1983, 1984a; Oldberg et al, 1983), rat plasma (Schwarzbauer et al, 1983). These data have tended to confirm that the basic FN polypeptide contains three different types of internal repeats (homology types I, II and III, approximatley 40, 60 and 90 amino acids long respectively, as originally shown in bovine plasma FN (Skorstengaard et al, 1982; Petersen et al, 1983). Variations on this basic fibronectin structure account for the differences between cellular and plasma fibronectins and also between the polypeptide chains of both forms.

The diverse forms of fibronectin seem to be generated by transcription of a single gene into a common precursor which undergoes alternative splicing (Vibe-Pedersen et al, 1984). To-date, at least two regions have been described where this type of variation occurs. In certain human cell lines (fibroblasts, Hs578T) FN mRNAs can be distinguished by a 270 nucleotide segment (ED) that encodes exactly one of the homology type III. This ED segment seems to be absent in the liver hepatocyte mRNAs which are the source of plasma FN (Kornblihtt et al, 1983, 1984b). Schwarzbauer et al (1983) have reported three different FN mRNAs arising by alternative splicing in rat liver which differ in an area (IIICS) located to the 3' carboxy terminus side in the protein of the ED region. The difference sequence does not belong to any of the known internal homologies and it is inserted between the last two type III homology repeats, near the COOH terminus. In addition, Umezawa et al (1985) have reported further variations in the equivalent IIICS area of human liver FN mRNA, bringing the total to 5 alternative motifs for this area. The differences observed between FN polypeptides are thus the consequence of internal primary sequence variability (Kornblihtt et al, 1984a 1984b; Schwarzbauer et al, 1983), due to alternative splicing in at least two distinctive regions of the pre-mRNA (Tamkun et al, 1984; Vibe-Pedersen et al, 1984; Umezawa et al, 1985).

The complete amino acid sequence of mature human FN polypeptides has now been determined from the nucleotide sequence of multiple cDNA clones as described below. The polypeptide length varies from 2146 to 2325 amino acids, depending on which internal alternative splicing has taken place.

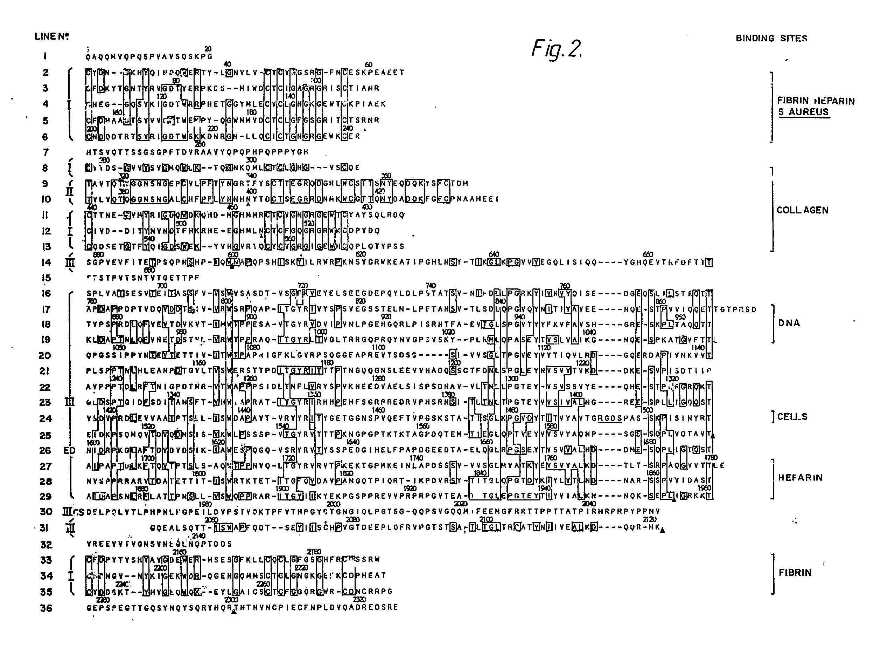

The present invention thus makes it possible to provide any desired part of the fibronectin molecule and in particular polypeptides having each of the separate binding activities of fibronectin separate from the others. In the accompanying Figure 2, the binding sites of each part of the FN amino-acid sequence are given. While some of these were previously known, the sequence for the human collagen-binding site (lines 8—13 in the Figure) is new. Lines 9 and 10, involving the type II homology, are believed to be of particular significance, and to incorporate most or all of the collagen-binding ability.

The present invention thus provides novel polypeptides having substantially the amino acid sequence from 379 to 455 shown in Figure 2, or any continuous part thereof having collagen-binding activity. In practice such polypeptides may be linked to further amino-acid residues not affecting the desired end use of the collagen-binding polypeptide, including additional residues of the fibronectin molecule itself.

Similarly other sequences of the fibronectin molecule may be utilized for their ability to bind to other molecules. Thus, as shown in Figure 2, the polypeptide sequence from 21 to 241 is associated with binding to fibrin, heparin and Staphylococcus aureus. Other sequences are, as shown, associated with binding to DNA, cells and alternative heparin and fibrin binding sites.

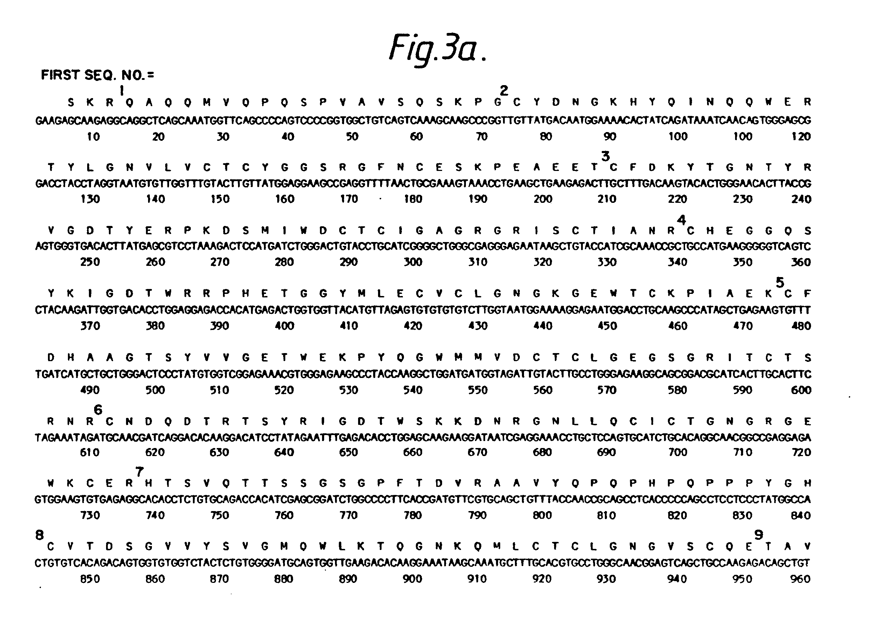

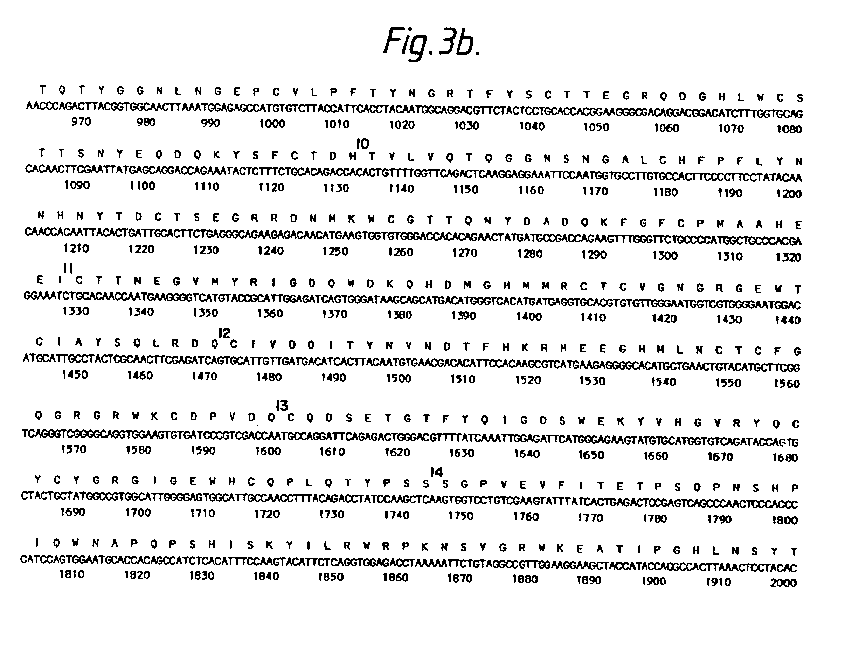

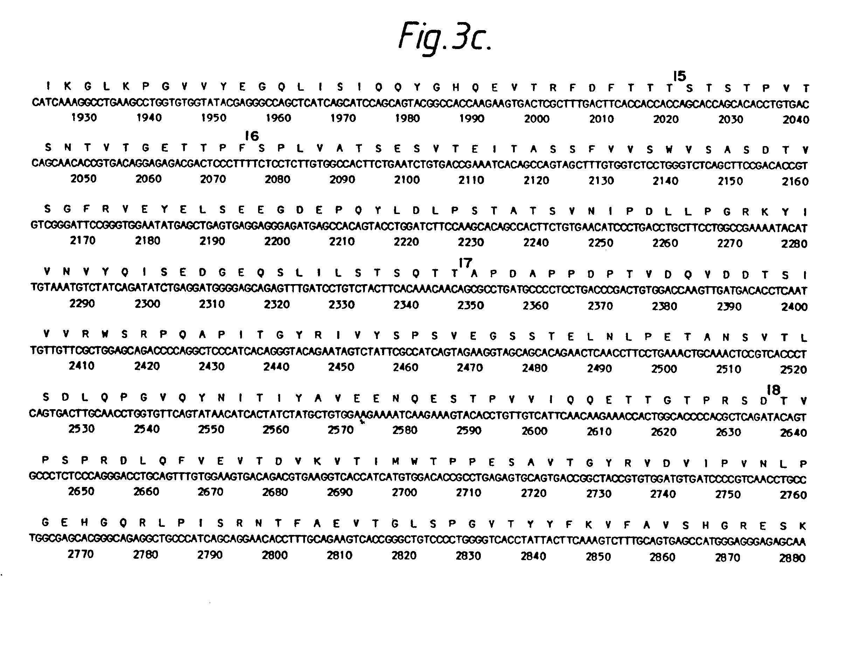

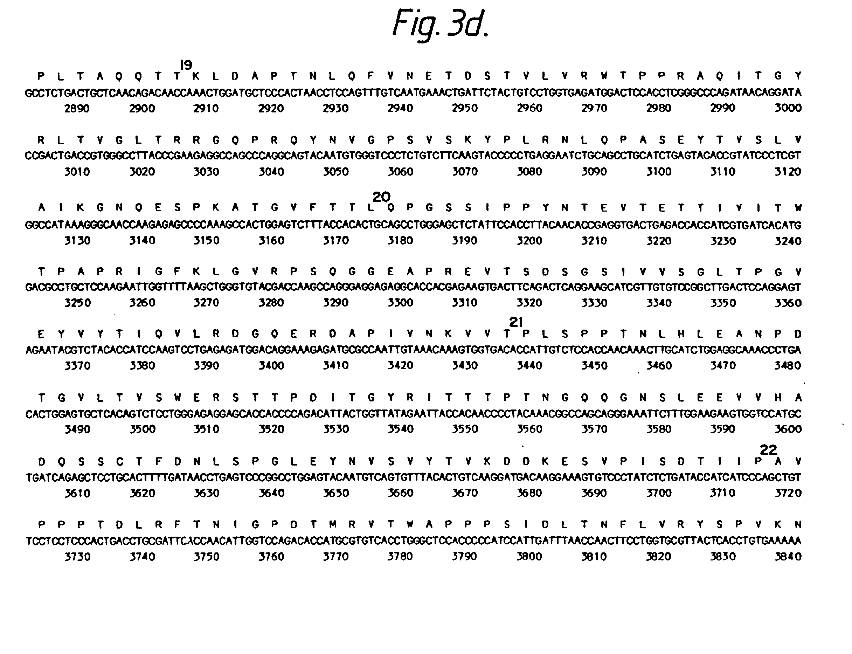

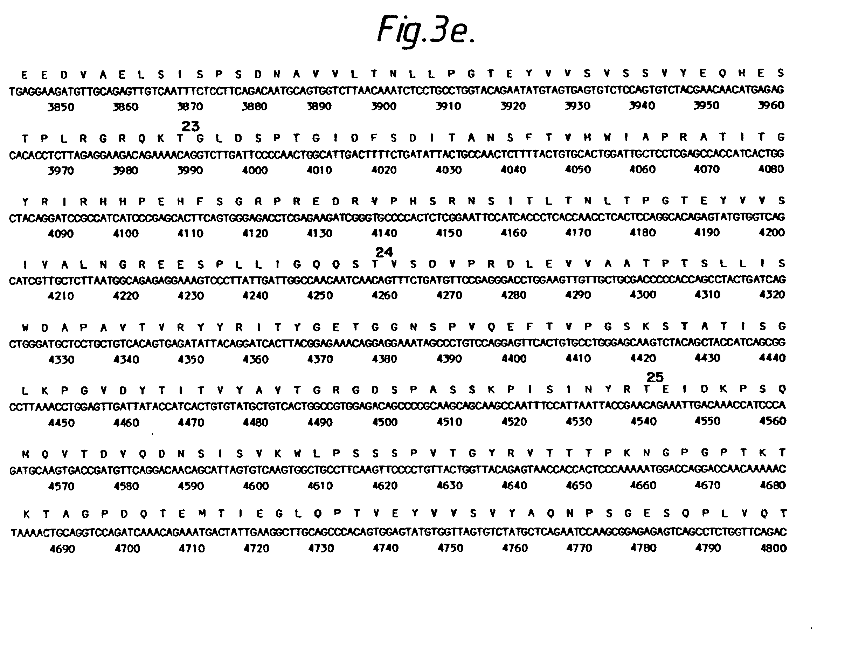

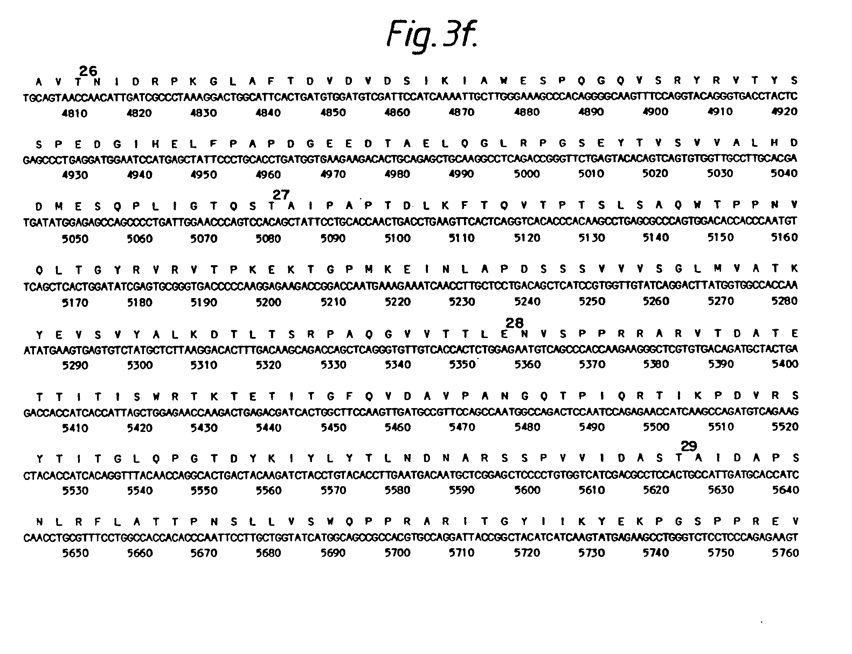

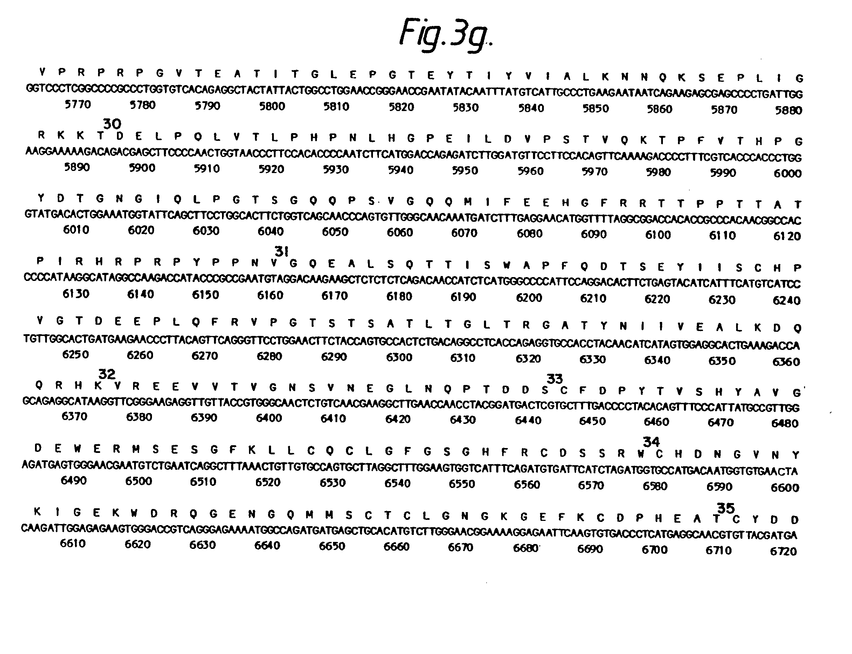

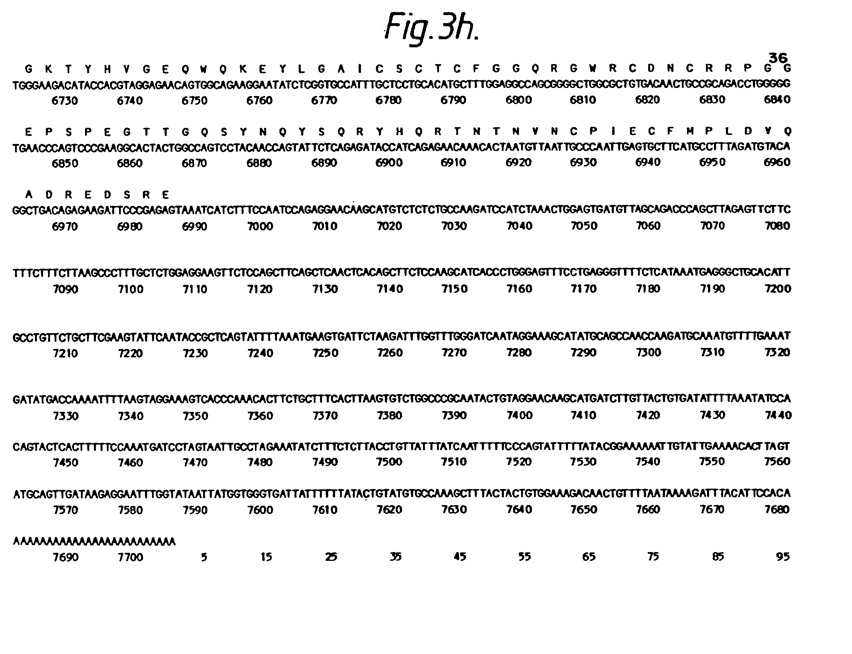

The novel polypeptides may be made by culturing cells containing endogenous DNA coding for the polypeptide and separating the polypeptide from the metabolic products. Thus, the appropriate DNA sequences may be cloned into a competent strain of E. coli or other microorgganism, e.g. a yeast such as Saccharomyces cerevisiae, the latter cultivated, and then the desired polypeptide isolated from the cultivation products. Figure 3 shows the complete DNA sequence for fibronectin and the associated amino-acid residues, and from this the cNDA sequence required for cloning the expression of any desired part of the fibronectin molecule may be easily determined.

The DNA sequence coding for the collagen-binding polypeptide runs from coordinate 1147 to coordinate 1351, and the sequence coding for the fibrin-binding polypeptide runs from coordinate 73 to 738.

More particularly, however, in accordance with the present invention, the clones herein described as pFH54, pFH134, pFH16 and pFH6 and similar clones may be used to produce corresponding polypeptides by expression in *E. coli* or other appropriate microorganisms. pFH134 and pFH16 contain the DNA sequence for the collagen-binding part of the fibronectin molecule and may be used to generate a polypeptide having the collagen-binding activity of fibronectin without its other binding affinities. pFH6 can be used to transform competent *E. coli* for the expression of a polypeptide binding fibrin and heparin.

The manner in which the clones pFH54, pFH134, pFH16 and pFH6 were obtained is described below, and repetition of the methods referred to will give similar clones with essentially the same or only slightly different utility. It will be appreciated in this connection that the isolation of useful cDNA sequences using the total cellular RNA from an appropriate source, i.e. cells naturally capable of expressing the desired protein or polypeptide, is a matter of routine experimentation for the person skilled in the art using the currently available techniques, particularly when, as is the case here, the actual amino acid sequence for the desired protein or polypeptide and the corresponding DNA sequence are both known. The experimental section below mentions appropriate techniques which have been found to be effective but it is to be expected that other known techniques would be equally applicable. Similarly choice of appropriate sources of RNA, vectors, and competent microorganisms for transformation from the many materials which are now available to the experimentalist is well within the ordinary skill in this art.

It will be appreciated that when a desired amino-acid sequence of fibronectin is expressed by a transformed microorganism it may be associated with a polypeptide characteristic of the microorganism itself. This may be immaterial to the intended use of the polypeptide but, in some cases, e.g. if the polypeptide is to be used in therapy, the presence of the additional amino-acid residues may be unacceptable. In that case the polypeptide must be subjected to an additional treatment, e.g. with a protease, to separate the desired polypeptide free from undesired additional amino-acid residues.

As indicated above, the present invention is of especial interest in that it provides a means for transforming an appropriate microorganism to make it capable of expressing a polypeptide able to bind to collagen and/or fibrin. A polypeptide able to bind to collagen can be used, for example, to facilitate affinity purification of valuable polypeptides. Thus if the collagen-binding polypeptide is expressed in a form in which it is bound to another polypeptide of interest or if it is linked, after isolation, to such a polypeptide, the combined polypeptides may be purified by affinity chormatography on a column of bound gelatin (i.e. collagen), and then, after the purification, the desired polypeptide may be separated, e.g. by an enzymatic hydrolysis, from the collagen-binding polypeptide.

A polypeptide able to bind to fibrin may be used in therapy to target a therapeutic agent on natural fibrin, e.g. a blood clot. For example, a fibrinolytic enzyme bound to the polypeptide would have improved clot-dissolving properties, since it would have improved adhesion to its target.

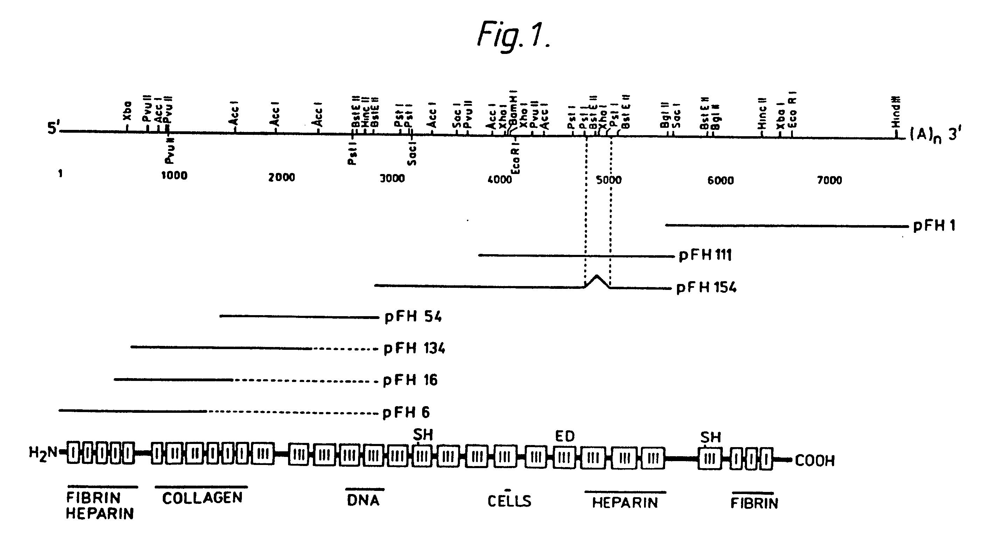

In the accompanying drawings, *Fig. 1* shows a restriction enzyme map of seven cDNA clones covering 7692 nucleotides from the poly(A) tail of human FN mRNA. Human FN mRNA has been estimated to be 7900 nucleotides long (Kornblihtt *et al*, 1983). The clones cover the complete coding region for the mature protein (bottom diagram showing binding sites) and the 3' non-coding region. The dotted lines indicate segments that are absent from the corresponding cDNA clones but that must have been synthesized in the first strand cDNA reaction and that were lost as a result of the failure of the Klenow enzyme to complete the second cDNA strand. Numbering on the map is in base pairs.

*Fig. 2* shows the complete amino acid sequence of a human FN polypeptide. Residues 1 and 2325 are the $NH_2$ and COOH termini of the mature protein. The sequence was deduced from the nucleotide sequence (see Figure 3) of the cDNA clones depicted in Fig. 1. Alignment shows internal homologies. Gaps were introduced to maximize the homology. Identical residues within a type of homology are boxed. The cell recognition tetrapeptide RGDS (Pierschbacher & Ruoslahti, 1984) is underlined. Positions 17 (Ser), 21 (Cys) and 42 (Val) are reported as Cys, Ser and Ala respectively by Garcia-Pardo *et al* (1983). The FN polypeptide shown in this Figure has 2325 residues with a MW=255,905. If the mass contributed by the carbohydrate side chains, estimated to be 9% of the protein mass (Yamada, 1983), is added, the molecular weight of this FN polypeptide would increase to approximately 279,000. This figure appears to be considerably higher than the weights of the FN monomers estimated by SDS-PAGE (230—250,000). The discrepancy could be explained by the poor resolution of the SDS gels in the range of high molecular weight proteins together with the lack of appropriate protein standards in that range.

Symbols are as follows: ■, free SH groups, λ, sites for carbohydrate side chains; Δ, cleavage site for chymotrypsin, ▲, cleavage site for plasmin. The multiple fibronectin polypeptides can be generated by all the possible permutations of the alternative splice regions in lines 26 and 30 (as explained below).

*Fig. 3* shows the complete nucleotide sequence for the human FN polypeptide of Figure 2 deduced from the sequences of the cDNA clones of f Figure 1.

*Fig. 4* shows the variations of the FN primary structure.

A is the complete structure of the mature protein. Black boxes are type I homologies; dashed boxes, type II homologies; empty boxes, type III homologies.

B and C show schematic representation of the different FN polypeptides that could arise by the translation of the multiple FN RNAs observed in the ED region (B) and in the IIICS region (C). The name of the cDNA clones representing the corresponding encoding mRNA species is shown to the right of each polypeptide. ∧ indicates contiguity. λr1f2, 4 and 6 were isolated from a rat liver cDNA library (Schwarzbauer et al, 1983), pFHL1 and 8 from a human liver cDNA library, and pFH1 was isolated from the Hs578T cell line cDNA library (Kornblihtt et al, 1983). It will be noted that all the variations include the zones binding to collagen and fibrin.

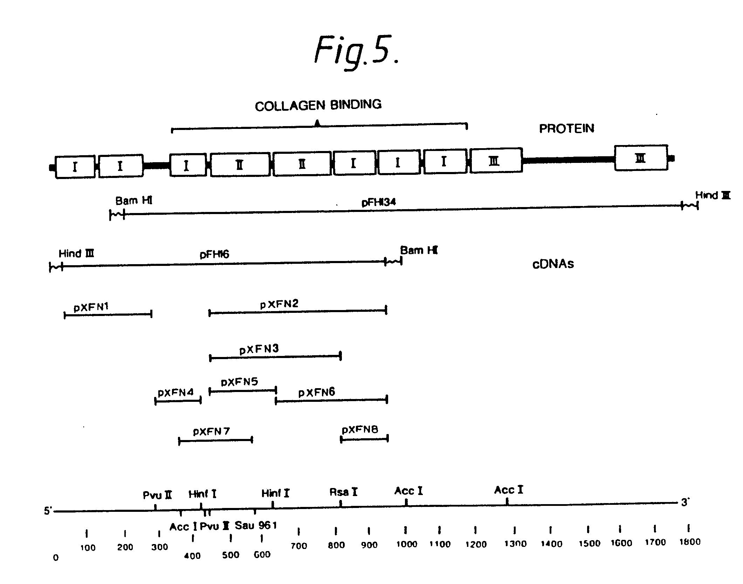

Figure 5 shows, in more detail than Figure 1, part of the fibronectin protein indicating the position of the collagen-binding domain and internal homologies (I, II, and III). Below, the position and sizes (in base pairs) of the series of cDNAs used in the bacterial expression experiments described below is indicated. Only the restriction enzyme sites relevant to the cDNAs pXFN1—8 are shown. The flanking Hind III and Bam HI sites of pFH134 and pFH16 occur in the polylinker of the vector.

Figure 1 shows the restriction map of different cDNA clones covering the 3' non-coding region and the complete coding region for the mature protein of human FN mRNA. The isolation of clones pFH1, pFH111 and pFH154 has been previously described (Kornblihtt et al, 1983, 1984a) and nucleotide sequence and deduced amino acid sequence of the latter has been published before (Kornblihtt et al, 1984b). The clones pFH54, pFH134, pFH16 and pFH6 are new. Isolation of these four cDNA clones covering the 5' third of the map involved the synthesis of an oligonucleotide primer. The sequence (namely 5-GCTGAACCATTTGCTGAGC) of the primer was complementary to the mRNA sequence of a region close to the 5' end of clone pFH154. The oligonucleotide was used to prime reverse transcription of total RNA from Hs578T cells (Hackett et al, 1977) and a cDNA library was prepared as described below. The clones pFH54, pFH134, pFH6 and pFH16 were selected for further analysis. The complete nucleotide sequence of these clones was determined and comprised 7692 bp, of which 6972 bp correspond to the coding region and 720 bp to the 3' nontranslatable region and poly(A)tail. The sequence is included in the full DNA sequence for FN shown in Figure 3.

The amino acid sequence of human fibronectin deduced from the nucleotide sequence of the clones in Figure 1 is shown in Figure 2. The alignment in Figure 2 maximises internal homologies. The complete FN chain presents regions which have 3 different types of internal homologies (types I, II and III) (Petersen et al, 1983) and regions which have no homologous counterparts within the molecule. The latter are the NH₂-terminal and COOH-terminal segments and the internal connecting strands. From NH₂- to COOH- terminus, FN is formed by one 20-residue long NH₂-terminal segment (Fig. 2, line 1), five units of type I homology or fingers (lines 2 to 6), one connecting strand (line 7), one finger (line 8), two units of type II homology (lines 9 and 10), three fingers (line 11, 12 and 13), one unit of type III homology (line 14), one connecting strand (line 15), fourteen units of type III homology (lines 16 to 29, including th ED polypeptide), one connecting strand (IIICS, line 30), one unit of type III homology (line 31), one connecting strand (line 32), three fingers (lines 33, 34 and 35) and the COOH-terminal segment (line 36).

The primary structure of FN reflects a level of order and complexity not seen before in any other protein. The symmetry in the array of the 16 units of type III homology is particularly interesting. Two type III units (Fig. 2, lines 14 and 31) are separated by connecting strands (lines 15 and 30) from a central block, having the remaining fourteen in a juxtaposed way. The degree of homology within the type III units is very high. Three residues are conserved in all the units, viz Trp (Fig. 2, box with residue 599 at the top), Leu (box with residue 640 at the top) and Tyr (box with residue 646 at the top). The conserved residues are distributed in two peaks around the Trp and the Tyr, separated by a valley of non homology. It is believed that the degree of order and conservation in the type III sequences must reflect particular constraints of the secondary structure of the central region of FN. This region is not stabilized by disulphide bridges since the only two Cys residues present in the type III sequences (positions 1201 and 2075 in Figure 2) have been shown to exist in a reduced form (Vibe-Pedersen et al, 1982; Smith et al, 1982).

Several binding activities have been assigned to different regions of the FN molecule (see Figs. 1 and 2). However, only in the case of the ability to bind cells, has the actual binding site been identified so far. In fact, Pierschbacher and Ruoslahti (1984) demonstrated that the tetrapeptide Arg-Gly-Asp-Ser (RGDS) is responsible for the cell attachment activity of FN. This tetrapeptide is present only once in the sequence of Fig. 2 at positions 1493 to 1496, within one of the type III units. Fig. 2 also shows that the optimal alignment of the type III sequences in this area is obtained only if the tetrapeptide is considered to be an extra element, allowing four gaps in the corresponding regions of the rest of the type III units. It is probable that, as well as the cell binding site, other binding sites or biological activities within type III sequences reside in non-conserved stretches. The tetrapeptide has also been found in other proteins (Pierschbacher & Ruoslahti, 1984) including the α chain of fibrinogen which shows cell attachment activity.

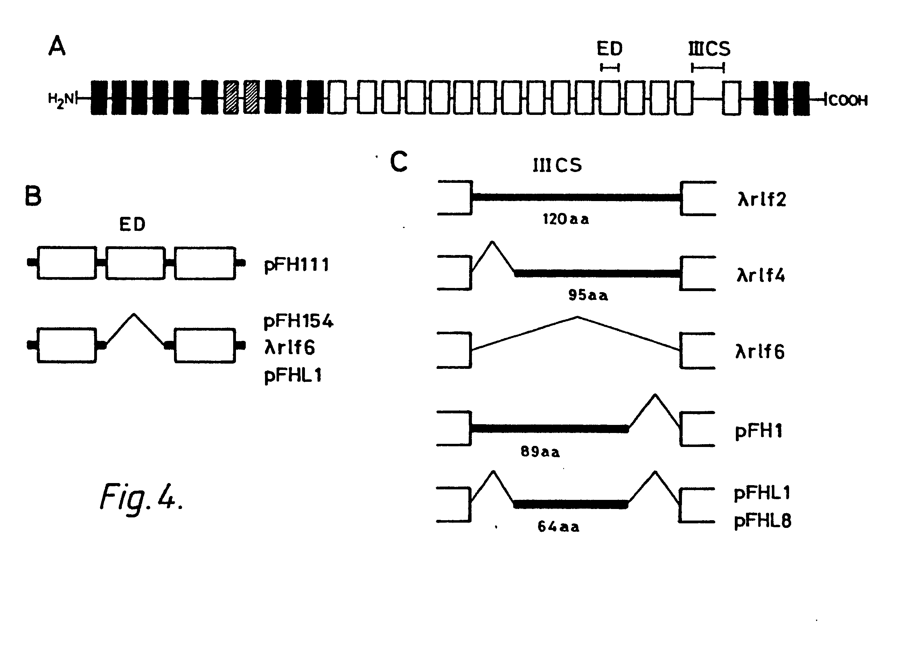

An important feature of FN gene expression is the generation of slightly different polypeptides by differential processing of the common mRNA precursor (Vibe-Pedersen et al, 1984). Figure 4A shows diagrammatically the localization of the two regions of variability observed so far along the FN molecule (Schwarzbauer et al, 1983; Kornblihtt et al, 1984a, 1984b). Figures 4B and 4C show the types of polypeptide that can arise from the translation of the different mRNAs generated in the ED (Fig. 2 line 26) and IIICS (Fig. 2, line 30) regions respectively. This diagram combines observations made on both human and rat fibronectin. At least 10 different FN polypeptides can be generated from a single gene if it be assumed that all the permutations between the ED and IIICS segments are possible. This is consistent with the FN polypeptide heterogeneity observed in the bidimensional gel electrophoresis analysis of cellular and plasma FNs found *in vivo*. Homo- or hetero- dimeric FN molecules can then be formed form the FN polypeptide pool. The biological significance of this complex situation is not yet clear. However, it is to be noted that the ED and IIICS variable regions are intercalated between the cell-heparin and heparin-fibrin binding sites. The distance between these biologically active sites of the molecule may be critical for the FN function. For example, plasma FN is 1 to 2 orders of magnitude less active than cellular fibronectin in restoring morphology and alignment to a transformed fibroblast cell line (Yamada & Kennedy, 1979). Further the mRNAs carrying the ED segment are present in fibroblasts (one source of cellular FN) but not in liver cells (one source of plasma FN) (Kornblihtt *et al*, 1984b). It is possible that the function of the ED is to increase the distance between the cell binding tetrapeptide and the heparin binding site, resulting in an enhanced binding activity of the cellular FN molecule.

Experimental
RNA Preparation

Human cell line Hs578T (Hackett *et al*, 1977) was cultured in Dulbecco's modified Eagle's medium containing 10% foetal calf serum. Total RNA was extracted from confluent cell monolayers by the guanidine-HCl method (Chirgwin *et al*, 1979). Between 2 and 4 mg of total RNA were extracted from $4 \times 10^8$ cells.

Other sources of RNA could be used if preferred, e.g. fibroblasts or liver cells.

Isolation of fibronectin cDNA clones

All the cDNA clones depicted in Figure 1 were obtained using Hs578T cell RNA as template. Isolation of clone pFH1 by oligonucleotide probing was described by Kornblihtt *et al* (1983). Isolation of clones pFH111 and pFH154 by "mRNA walking" (oligonucleotide priming) was described by Kornblihtt *et al* (1984a). This latter procedure was used for the isolation of the new clones pFH54, pFH134, pFH16 and pFH6. An oligonucleotide primer complementary to the mRNA region close to the 5' end of pFH154 was synthesized by the method of Gait *et al* (1980). The oligonucleotide was used to prime reverse transcription of total RNA from Hs578T cells (Hackett *et al*, 1977). Blunt ended ds cDNA was prepared and cloned into the plasmid pAT153/Pvull/8 (Anson *et al*, 1984) in *E. coli* MC1061 as previously described (Kornblihtt *et al*, 1983). Colonies were screened using as probe a restriction fragment from the 5' end of pFH154 lacking the primer sequences, labelled by filling in at one end. In this way, clones pFH54 and pFH134 were obtained. In a second step, clones pFH16 and 6 were obtained by screening with an end labelled probe for the 5' end of clone pFH134. Restriction fragments of the fibronectin cNDA were filed in with the Klenow fragment of DNA polymerase I and blunt end ligated into SmaI cut/phosphatased pEX 1, 2 or 3 vector. Transformations were carried out using the *E. coli* strain LKIII (Zabeau *et al*, 1982) harbouring the plasmid pc1857 which specifies kanamycin resistance and carries the c1857 allele (Remaut *et al*, 1983). Colonies were transferred to Whatman 541 filter paper (Gergen *et al*, 1985) and screened with either 3' end labelled (Maxam *et al*, 1977) or nick translated probes (Rigby *et al*, 1977).

Sequence determination

Inserts from clones were excised from the vector DNA by digestion with appropriate restriction enzymes, separated in agarose gel electrophoresis, and recovered by electroelution (Girwitz *et al*, 1980). Most of the sequencing was performed by the chemical degradation procedure of Maxam and Gilbert (1980). Some regions were sequenced by the chain terminator method (Sanger *et al*, 1977). For that purpose, the relevant fragments were isolated, digested either with *AluI* or *HaeIII* and ligated to a *SmaI* digested M13mp9 vector (Messing & Vieira, 1982), previously treated with calf intestinal phosphatase to prevent its circularization. The ligation mixtures were used to transform competent *E. coli* JM101 and recombinants were selected as clear plaques by insertional inactivation of the β-galactosidase gene (Messing *et al*, 1981). Single stranded DNA was prepared by standard procedures (Winter & Fields, 1980) and the inserts were sequenced using a "universal" 17-nucleotide long primer (Duckworth *et al*, 1981).

Preparation of bacterial extracts

Bacteria carrying recombinant plasmids were grown at 30°C for 2½ h and expression of the cro/β-galactosidase fusion protein induced by shifting to 42°C for 2h. Bacteria were pelleted at 1200 g and washed with 50 mM TrisHCl, pH 7.4, 170 mM NaCl. Cells were resuspended in the same buffer containing lysozyme (2.5 mg/ml) and sonicated for 2 min on ice. The lysate was centrifuged at 45,000 g for 30 min at 4°C. The pellet was resuspended in 7 M urea in 10 mM Tris HCl, pH 7.4, 1mM EDTA and incubated at room temperature for 30 min. The solubilised extract was dialysed extensively against 50 mM Tris HCl pH 7.4 at 4°C, and then centrifuged at 45,000 g for 30 min at 4°C.

Gelatin-Sepharose chromatography

Gelatin-Sepharose was either obtained from Sigma Chemicals (St. Louis, MO, USA) or prepared by linking gelatin (pig skin type I, Sigma Chemicals) to CNBr-activated Sepharose CL.4B (Pharmacia, Uppsala, Sweden). Chromatography of bacterial extracts on gelatin-Sepharose was carried out as described by Ruoslahti *et al* (1982). The efficacy of the gelatin-Sepharose matrix was verified using purified human plasma fibronectin (Sigma Chemicals).

Electrophoretic analysis

SDS-polyacrylamide gel electrophoresis was carried out in 0.1% (w/v) SDS in Tris/glycine buffer on 7.5% (w/v) acrylamide slab gels (19). Gels were stained with 0.1% Coomassie blue in methanol/water/acetic acid (4:5:1 by vol.). Immunoblotting was performed as described by Towbin et al. (1979). Polypeptides, electrophoretically transferred to nitrocellulose were probed with rabbit anti(human plasma fibronectin) serum (1:500 in phosphate buffered saline, 10% newborn calf serum and 0.05% Tween 20). Bound immunoglobulin was visualised using alkaline phosphatase conjugated goat anti-(rabbit IgG) (1:1000; Sigma Chemicals).

Protein assay

Protein was estimated by the method of Bradford (1976) using bovine serum albumin as a standard.

Construction and characterisation of fibronectin expression plasmids

The human fibronectin cDNA clones, pFH134 and pFH16, encompass all or part of the collagen-binding domain of fibronectin identified by proteolytic cleavage of the protein (see Fig. 1 and Fig. 5). These cDNAs were therefore chosen as the starting point for investigating the expression of a functional collagen-binding site in *E. coli*. The pEX vectors used for cloning enable exogenous gene sequences to be inserted into a polylinker in all three reading frames at the 3′ end of a cro-LacZ hybrid gene under the control of the $\lambda P_r$ promoter (Stanley *et al*, 1984). The 5′ ends of the 1.74 kb and 1.04 kb inserts of pFH134 and pFH16 respectively were sequenced (Maxam *et al*, 1980) to establish the reading frames of the cDNAs and blunt end cloned into the *Sma*I site of pEX2. The recombinant plasmids were introduced into an *E. coli* strain previously transformed with a plasmid encoding the temperature-sensitive $\lambda P_r$ repressor, c1857. This allows for temperature-inducible expression of the cro/β-galactosidase protein. To test for the production of fibronectin fusion protein by the expression constructs, hybridisation positive clones were grown at 30°C for $2\frac{1}{2}$ h and then shifted to 42°C for a further 2 h. Total bacterial lysates were analysed by SDS polyacrylamide gel electrophoresis. Five of ten pXFH134 constructs and one of seven pXFH16 constructs showed the production of high molecular weight polypeptides of sizes consistent with the lengths of the cDNA inserts (~185 kD and ~165 kD respectively. The correct orientation of the fibronectin sequences in pXFH134 and pXFH16 was confirmed by restriction enzyme anlayses.

The fusion proteins produced by pXFH134 and pXFH16 accounted for approximately 20% of the total bacterial protein consistent with the previous report for this vector system (Stanley *et al*, 1984). Both fusion proteins showed some proteolytic degradation, particularly the pXFH134 polypeptide, which appeared to be partially cleaved to the size of the wild-type cro/β-galactosidase (116 kD). Analysis of proteins synthesised over a time course of induction (0 to 120 min) indicated that proteolysis occurred concomitantly with synthesis of the fusion proteins.

The expression of fibronectin antigenic determinants in pXFH134 and pXFH16 was investigated by immunoblotting using a rabbit polyclonal anti-(human plasma fibronectin) serum. The antiserum reacted with the 185 kD polypeptide synthesised by pXFH134 but not with the pXFH16 fusion protein or the cro/β-galactosidase polypeptide, indicating that the epitope(s) recognised by the anti-serum lie outside the type II homology units and adjacent type I repeats (Fig. 5). This observation is consistent with the poor antigenicity of the collagen-binding domain of human fibronectin previously reported (Ruoslahti *et al*, 1979) and most probably reflects the very high level of amino acid conservation in this region (Fig. 2).

Gelatin-Sepharose affinity chromatography

Over-production of β-galactosidase fusions in *E. coli* results in the precipitation of the protein in the cells as insoluble inclusion bodies (Williams *et al*, 1982, Cheng 1983, Stanley 1983). Thus, when bacteria expressing the pXFH134 plasmid were lysed by sonication and centrifuged, the fibronectin fusion protein was found exclusively in the insoluble pellet. This fraction represented approximately 50% of the total protein of the bacterial lysate. Solubilisation of this material required treatment with 7 M urea and, following dialysis, 60% of the protein remained in solution. This fraction, which was highly-enriched in the fusion protein, was applied directly to a 5 ml gelatin-Sepharose column equilibrated in 50 mM Tris HCl, pH 7.4. The column was washed with 0.5 M NaCl in 50 mM Tris HCl, pH 7.4 until the $E_{280}$ of the flowthrough was <0.01. The cro/β-galactosidase-fibronectin hybrid protein was eluted from the column as a single symmetrical peak with 4 M urea in the same buffer. Under these conditions fibronectin is specifically released from gelatin-Sepharose (Ruoslahti *et al*, 1982). In the control experiment using pEX2 only, no binding of the wild-type cro/β-galactosidase protein was observed.

A functional collagen-binding site has therefore been reconstituted in the pXFH134 fusion protein. It must be noted, however, that the fusion protein specifically eluted from the column represented <5% of the fusion protein applied to the column. Thus, not surprisingly, considerable activity is lost due to the insolubilisation of the fusion proteins in the bacterial cells, and subsequent vigorous treatment required to resolubilise them.

The fibronectin fusion protein produced by pXFH16 was also tested for gelatin-binding and showed similar activity to pXFH134. This indicated that the collagen-binding region occurred within the domain defined at the protein level and strongly implicated the two type II and adjacent type I homology units (see Fig. 5). To further localise the binding site, a series of overlapping expression constructs was made from pFH16 (Fig. 5) and systematically assayed for gelatin-binding activity. The results are summarised in Table 1, and show the consistent involvement of the type II homology units (pXFN 2, 3 and 6).

TABLE 1

| pEX construct | Binding to gelatin-sepharose |
|---|---|
| pXFH134 | + |
| pXFH16 | + |
| pXFN1 | − |
| 2 | + |
| 3 | + |
| 4 | − |
| 5 | − |
| 6 | + |
| 7 | − |
| 8 | − |
| pEX2 vector only | − |

The binding activity of pXFH134 is almost entirely accounted for by a construct consisting of the two type II homology units (pXFN3). By comparing the gelatin-binding activity of pXFN3 and pXFN6 (both active) with pXFN5 and pXFN8 (both inactive), it may be deduced that the amino acid sequence critical for binding lies in the C-terminal half of the fibronectin fragment in pXFN3, and more particularly from the HinfI site (coordinate 1147 of Figure 3) to the RsaI site (coordinate 1351 of Figure 3). This 66 amino acid sequence represents almost the entire second type II homology unit of fibronectin plus a few amino acids of the adjacent type I homology unit (see Fig. 2).

References

Anson, D., Choo, K. H., Rees, D. J. G., Giannelli, F., Gould, K., Huddleston, J. A. and Brownlee, G. G. (1984) EMBO J. 3, 1053—1064.

Bradford M. (1976). Anal. Biochem., 72, 248—254.

Chirgwin, J. M., Przybyla, A. E., MacDonald, R. J. and Rutter, W. J. (1979) Biochemistry 18, 5294—5299.

Cheng Y. (1983), Biochem. Biophys, Res. Commun., 111, 104—111.

Duckworth, M. L., Gait, M. J., Goelet, P., Hong, G. F., Singh, M. and Titmas, R. C. (1981) Nucl. Acids Res. 9, 1691—1706.

Gait, M. J. et al (1980) Nucl. Acids Res. 8, 1081—1096.

Garcia-Pardo, A., Pearlstein, E. and Frangione, B. (1983) J. Biol. Chem. 258, 12670—12674.

Gergen J. P., Stern R. H., and Wersink P. C. (1979), Nucl. Acids Res. 7, 2115—2136.

Girwitz, S.C., Bacchetti, S., Rainbow, A. J. and Graham, F. L. (1980) Anal. Biochem. 106, 492—496.

Hackett, A. J., Smith, H. S., Springer, E. L., Owens, R. B., Nelson-Rees, W. A., Riggs J. L. and Gardner, M. B. (1977) J. Natl. Cancer Inst. 58, 1795—1800.

Hynes, R. O. and Yamada, K. M. (1982) J. Cell. Biol. 95, 369—377.

Kornblihtt, A. R., Vibe-Pedersen, K. and Baralle, F. E. (1983) Proc. Natl. Acad. Sci. USA 80, 3218—3222.

Kornblihtt, A. R., Vibe-Pedersen, K. and Baralle, F. E. (1984a) EMBO J. 3, 221—226.

Kornblihtt, A. R., Vibe-Pedersen, K. and Baralle, F. E. (1984b) Nucl. Acids Res. 12, 5853—5868.

Maxam, A. M. and Gilbert, W. (1977) Proc. Natl. Acad. Sci. U.S.A. 74, 560—569.

Maxam, A. M. and Gilbert, W. (1980) Meth. Enzym. 65, 499—580.

Messing, J. and Vieira, J. (1982) Gene 19, 269—276.

Messing, J., Crea, R. and Seeburg, P. H. (1981) Nucl. Acids Res. 9, 309—321.

Oldberg, A., Linney, E. and Ruoslahti, E. (1983) J. Biol. Chem. 258, 10193—10196.

Pande, H. and Shively, J. E. (1982) Arch. Biochem. Biophys. 213, 258—265.

Petersen, T., Thøgersen, H. C., Skorstengaard, J, K., Vibe-Pedersen, K., Sahl, P., Sottrup-Jesen, L. and Magnusson, S. (1983) Proc. Natl. Acad. Sci. USA 80, 137—141.

Pierschbacher, M. D. and Ruoslahti, E. (1984) Nature 309, 30—33.

Remaut E., Tsao H, and Fiers W. (1983) Gene, 22, 103—113.

Rigby P. W. J., Dieckmama H. Rhodes C., and Berg P. (1977) J. Mol. Biol. 113, 237—251.

Ruoslahti, E., Hayman, E. G., Kinnsela P., Shively J. E., and Engvall E., (1979), J. Biol. Chem. 254, 6054—6059.

Ruoslahti, E., Hayman E. G., Piersbacher M., and Engvall E. (1982), Meth. Enzymol. 82, 803—831.

Sanger, F., Nickeln S. and Coulson, A. R. (1977) Proc. Natl. Acad. Sci. USA 74, 5463—5467.

Schwarzbauer, J. E., Tamkun. J. W., Lemischka, I. R. and Hynes, R. O. (1983) Cell 35, 421—431.

Skorstengaard, K., Thøgersen, H. C., Vibe-Pedersen, K., Petersen, T. E. and Magnusson S., (1982) Eur. J. Biochem, 128, 605—623.

Smith, D. G., Mosher, D. F., Johnson, R. B. and Furcht, L. T. (1982) J. Biol. Chem. 257, 5831—5838.

Stanley K. K. (1983), Nucl. Acids Res. 11, 4077—4092.

Stanley K. K. and Luzio, P. (1984) EMBO J. 3, 1429—1434.

Tamkun, J. K., Schwarzbauer, J. E. and Hynes, R. O. (1984) Proc. Natl. Acad. Sci. USA 81, 5140—5144.

Towbin H., Staehlin T. and Gordon J. (1979), Proc. Natl. Acad. Sci. U.S.A. 76, 4350—4354.

Umezawa, K., Korblihtt, A. R. and Baralle, F. E. (1985) submitted to FEBS Lett.

Vibe-Pedersen, K., Sahl, P., Skorstengaard, K.

and Petersen, T. E. (1982) FEBS Lett. *142*, 27—30.

Vibe-Pedersen, K., Kornblihtt, A. R. and Baralle, F. E. (1984) EMBO J. *3*, 2511—2516.

Williams D. C., Van Frank R. M., Muth W. L. and Burnett J. P. (1982), Science *215*, 687—689.

Winter, G. and Fields, S. (1980) Nucl. Acids Res. *8*, 1965—1974.

Yamada, K. M. and Kennedy, D. W. (1979) J. Cell. Biol. *80*, 492—498.

Yamada, K. M. (1983) Ann. Rev. Biochem. *52*, 761—799.

Zabeau M. and Stanley K. K. (1982) EMBO J., *1*, 1217—1224.

**Claims for the Contracting States: BE, CH, DE, FR, GB, IT, LI, LU, NL, SE**

1. A polypeptide sequence having specific affinity for collagen or fibrin, wherein the collagen-binding sequence is the amino-acid sequence 379 to 445 shown in Figure 2 or a continuous collagen-binding part thereof and the fibrin-binding portion is the amino-acid sequence 21 to 241 shown in Figure 2 or a continuous fibrin-binding part thereof and in either case the said sequence is not colinear with and adjacent the amino acid sequences which are adjacent to and colinear with it in human fibronectin.

2. A polypeptide according to claim 1 bound to a polypeptide sequence not present in human fibronectin.

3. A method of purifying a substance which comprises contacting a conjugate of that substance and a collagen-binding sequence according to claim 1 with immobilized collagen so that the said conjugate binds to the said collagen, and then eluting the said conjugate.

4. A method according to claim 3 in which the said eluted conjugate is then split to remove the said collagen-binding sequence, and the said substance is then isolated.

5. A polypeptide comprising a fibrin-binding amino-acid sequence according to claim 1 bound to a therapeutic agent.

6. A cDNA sequence coding for a polypeptide sequence according to claim 1.

7. A cDNA sequence according to claim 6 having the structure shown at 1147 to 1351 in Figure 3b.

8. A cDNA sequence according to claim 6 having the structure shown at 73 to 738 in Figure 3a.

9. A plasmid or other vector containing a cDNA sequence as defined in any of claims 6 to 8.

10. A microorganism modified by inclusion of a vector as claimed in claim 9.

11. *Escherichia coli* modified by inclusion of a vector as claimed in claim 9.

**Claims for the Contracting State: AT**

1. A process for preparing a polypeptide sequence having specific affinity for collagen or fibrin, wherein the collagen-binding sequence is the amino-acid sequence 379 to 445 shown in Figure 2 or a continuous collagen-binding part thereof and the fibrin-binding portion is the amino-acid sequence 21 to 241 shown in Figure 2 or a continuous fibrin-binding part thereof and in either case the said sequence is not colinear with and adjacent the amino acid sequences which are adjacent to and colinear with it in human fibronectin, by expression of a nucleotide sequence.

2. A process according to claim 1 wherein the polypeptide as disclosed in claim 1 is bound to a polypeptide sequence not present in human fibronectin.

3. A method of purifying a substance which comprises contacting a conjugate of that substance and a collagen-binding sequence prepared according to claim 1 with immobilized collagen so that the said conjugate binds to the said collagen, and then eluting the said conjugate.

4. A method according to claim 3 in which the said eluted conjugate is then split to remove the said collagen-binding sequence, and the said substance is then isolated.

5. A process for preparing a polypeptide comprising a fibrin-binding amino-acid sequence according to claim 1 bound to a therapeutic agent, by binding the said amino acid sequence to the therapeutic agent.

6. A process for preparing a DNA sequence coding for a polypeptide sequence according to claim 1, by mRNA-directed DNA synthesis.

7. A process for preparing a DNA sequence according to claim 6 having the structure shown at 1147 to 1351 in Figure 3b.

8. A process for preparing a DNA sequence according to claim 6 having the structure shown at 73 to 738 in Figure 3a.

9. A process for preparing a plasmid or other vector containing a DNA sequence as defined in any of claims 6 to 8, by recombinant DNA techniques.

10. A method of transforming a microorganism by inclusion of a vector prepared as claimed in claim 9.

11. A method of transforming *Escherichia coli* by inclusion of a vector prepared as claimed in claim 9.

**Patentansprüche für die Vertragsstaaten: BE, CH, DE, FR, GB, IT, LI, LU, NL, SE**

1. Polypeptidsequenz mit spezifischer Affinität für Kollagen oder Fibrin, worin die Kollagen-bindende Sequenz aus der in Fig. 2 dargestellten Aminosäuresequenz 379 bis 445 oder einem fortlaufenden Kollagen-bindenden Teil derselben und der Fibrin-bindende Teil aus der in Fig. 2 dargestellten Aminosäuresequenz 21 bis 241 oder einem fortlaufenden Fibrin-bindenden Teil derselben bestehen und in jedem Falle die betreffende Sequenz nicht kolinear mit und benachbart zu den Aminosäuresequenzen, die in Humanfibronektin zu ihr benachbart und mit ihr kolinear sind, ist.

2. Polypeptid nach Anspruch 1, in an eine Polypeptidsequenz, die in Humanfibronektin nicht vorhanden ist, gebundener Form.

3. Verfahren zum Reinigen einer Substanz durch Inberührungbringen eines Konjugats der betreffenden Substanz und einer Kollagen-bindenden Sequenz nach Anspruch 1 mit immobilisiertem Kollagen zur Bindung des betreffenden Konjugats an das Kollagen und anschließendes Eluieren des Konjugats.

4. Verfahren nach Anspruch 3, bei welchem das eluierte Konjugat anschließend zur Entfernung der Kollagenbindenden Sequenz gespalten und die betreffende Substanz anschließend isoliert wird.

5. Polypeptid in Form einer an ein therapeutisches Mittel gebundenen Fibrin-bindenden Aminosäuresequenz nach Anspruch 1.

6. cDNA-Sequenz mit Kodierung für eine Polypeptidsequenz nach Anspruch 1.

7. cDNA-Sequenz nach Anspruch 6 der in Fig. 3b bei 1147 bis 1351 dargestellten Struktur.

8. cDNA-Sequenz nach Anspruch 6 der in Fig. 3a bei 73 bis 738 dargestellten Struktur.

9. Plasmid oder sonstiger Vektor mit einer cDNA-Sequenz nach einem der Ansprüche 6 bis 8.

10. Mikroorganismus, der durch Einschluß eines Vektors nach Anspruch 9 modifiziert ist.

11. Escherichia coli, das durch Einschluß eines Vektors nach Anspruch 9 modifiziert ist.

**Patentansprüche für den Vertragsstaat: AT**

1. Verfahren zur Herstellung einer Polypeptidsequenz mit spezifischer Affinität für Kollagen oder Fibrin, bei der die Kollagen-bindende Sequenz aus der in Fig. 2 dargestellten Aminosäuresequenz 379 bis 445 oder einem fortlaufenden Kollagen-bindenden Teil derselben und der Fibrin-bindende Teil aus der in Fig. 2 dargestellten Aminosäuresequenz 21 bis 241 oder einem fortlaufenden Fibrin-bindenden Teil derselben bestehen und in jedem Falle die betreffende Sequenz nicht kolinear mit und benachbart zu den Aminosäuresequenzen, die in Humanfibronektin zu ihr benachbart und mit ihr kolinear sind, ist, durch Expression einer Nukleotidsequenz.

2. Verfahren nach Anspruch 1, bei welchem das in Anspruch 1 erläuterte Polypeptid an eine in Humanfibronektin nicht vorhandene Polypeptidsequenz gebunden ist.

3. Verfahren zum Reinigen einer Substanz durch Inberührungbringen eines Konjugats der betreffenden Substanz und einer Kollagen-bindenden Sequenz nach Anspruch 1 mit immobilisiertem Kollagen zur Bindung des betreffenden Konjugats an das Kollagen und anschließendes Eluieren des Konjugats.

4. Verfahren nach Anspruch 3, bei welchem das eluierte Konjugat anschließend zur Entfernung der Kollagen-bindenden Sequenz gespalten und die betreffende Substanz anschließend isoliert wird.

5. Verfahren zur Herstellung eines Polypeptids mit einer an ein therapeutisches Mittel gebundenen Fibrin-bindenden Aminosäuresequenz nach Anspruch 1 durch Binden der betreffenden Aminosäuresequenz an das therapeutische Mittel.

6. Verfahren zur Herstellung einer DNA-Sequenz mit Kodierung für ein Polypeptid nach Anspruch 1 durch mRNA-gelenkte DNA-Synthese.

7. Verfahren zur Herstellung einer DNA-Sequenz nach Anspruch 6 der in Fig. 3b bei 1147 bis 1351 dargestellten Struktur.

8. Verfahren zur Herstellung einer DNA-Sequenz nach Anspruch 6 der in Fig. 3a bei 73 bis 738 dargestellten Struktur.

9. Verfahren zur Herstellung eines Plasmids oder sonstigen Vektors mit einer DNA-Sequenz nach einem der Ansprüche 6 bis 8 durch rekombinate DNA-Techniken.

10. Verfahren zur Transformation eines Mikroorganismus durch Einschluß eines gemäß Anspruch 9 hergestellten Vektors.

11. Verfahren zur Transformation von Escherichia coli durch Einschluß eines gemäß Anspruch 9 hergestellten Vektors.

**Revendications pour les Etats Contractants: BE, CH, DE, FR, GB, IT, LI, LU, NL, SE**

1. Séquence polypeptide ayant une affinité spécifique pour le collagène ou la fibrine, dans laquelle la séquence de liaison avec le collagène est la séquence d'acides aminés 379 à 445 représentée dans la Figure 2 ou bien une partie continue de cette séquence, formant une liaison avec le collagène, et la partie formant une liaison avec la fibrine est la séquence d'acides aminés 21 à 241 représentée dans la Figure 2 ou bien une partie continue de cette séquence, formant une liaison avec la fibrine, et dans l'un et l'autre cas ladite séquence n'est pas colinéaire ni adjacente aux séquences d'acides aminés qui sont adjacentes et colinéaires à elle dans la fibronectine humaine.

2. Polypeptide selon la revendication 1, lié à une séquence polypeptide non présente dans la fibronectine humaine.

3. Méthode de purification d'une substance comprenant la fait de mettre en contact un conjugué de cette substance et une séquence formant une liaison avec le collagène selon la revendication 1, avec du collagène immobilisé, de telle manière que ledit conjugué forme une liaison avec ledit collagène, et, ensuite, le fait d'éluer ledit conjugué.

4. Méthode selon la revendication 3, dans laquelle ledit conjugué élué est ensuite divisé pour enlever ladite séquence formant une liaison avec le collagène, et ladite substance est ensuite isolée.

5. Polypeptide comprenant une séquence d'acides aminés formant des liaisons avec la fibrine, selon la revendication 1, liée à un agent thérapeutique.

6. Séquence cDNA codante pour une séquence polypeptide selon la revendication 1.

7. Séquence cDNA selon la revendication 6

ayant la structure représentée en 1147 à 1351 dans la Figure 3b.

8. Séquence cDNA selon la revendication 6 ayant la structure représentée en 73 à 738 dans la Figure 3a.

9. Plasmide ou autre vecteur contenant une séquence cDNA telle que définie dans l'une quelconque des revendications 6 à 8.

10. Microorganisme modifié par inclusion d'un vecteur tel que revendiqué dans la revendication 9.

11. *Escherichia coli* modifié par inclusion d'un vecteur tel que revendiqué dans la revendication 9.

**Revendications pour l'Etat Contractant: AT**

1. Méthode de préparation d'une séquence polypeptide ayant une affinité spécifique pour le collagène ou la fibrine, dans laquelle la séquence de liaison avec le collagène est la séquence d'acides aminés 379 à 445 représentée dans la Figure 2, ou bien une partie continue de cette séquence, formant une liaison avec le collagène, et la partie formant une liaison avec la fibrine est la séquence d'acides aminés 21 à 241 représentée dans la Figure 2, ou bien une partie continue de cette séquence, formant une liaison avec la fibrine, et dans l'un et l'autre cas ladite séquence n'est pas colinéaire ni adjacente aux séquences d'acides aminés qui sont adjacentes et colinéaires à elle dans la fibronectine humaine, par expression d'une séquence nucléotide.

2. Méthode selon la revendication 1 dans laquelle le polypeptide visé dans la revendication 1 est lié à une séquence polypeptide non présente dans la fibronectine humaine.

3. Méthode de purification d'une substance comprenant le fait de mettre en contact un conjugué de cette substance et une séquence formant une liaison avec le collagène, préparée conformément à la revendication 1, avec du collagène immobilisé, de telle manière que ledit conjugué forme une liaison avec ledit collagène, et ensuite le fait d'éluer ledit conjugué.

4. Méthode selon la revendication 3, dans laquelle ledit conjugué élué est ensuite divisé pour enlever ladite séquence formant une liaison avec le collagène, et ladite substance est ensuite isolée.

5. Méthode de préparation d'un polypeptide comprenant une séquence d'acides aminés formant une liaison avec la fibrine selon la revendication 1, liée à un agent thérapeutique, par formation d'une liaison de ladite séquence d'acides aminés avec l'agent thérapeutique.

6. Méthode de préparation d'une séquence DNA codante pour une séquence polypeptide selon la revendication 1, par synthèse DNA dirigée par mRNA.

7. Méthode de préparation d'une séquence DNA selon la revendication 6, ayant la structure représentée de 1147 à 1351 dans la Figure 3b.

8. Méthode de préparation d'une séquence DNA selon la revendication 6, ayant la structure représentée de 73 à 738 dans la Figure 3a.

9. Méthode de préparation d'un plasmide ou autre vecteur contenant une séquence DNA telle que définie dans l'une quelconque des revendications 6 à 8, par les techniques de DNA recombinant.

10. Méthode de transformation d'un microorganisme par inclusion d'un vecteur préparé selon la revendication 9.

11. Méthode de transformation d'*Escherichia coli* par inclusion d'un vecteur préparé selon la revendication 9.

EP 0 207 751 B1

*Fig.1.*

Fig.2.

LINE N°

BINDING SITES

EP 0 207 751 B1

2

```
 1   QAQQHVQPQSPVAVSQSKPG
 2   CYDN---GKHYQIMPQWERTY-LGNVLV-CTCYWGSRG-FNCESKPEAEET
 3   LFDKYTGNTYRVGDTYERPKCS--MIWDCTCIGAGRGRISCTIANR
 4   GHEG--GQSYKIGDTWRRPHETGGYHLECVCLGNGKGEWTCKPIAEK
 5   CFDHAALTSYVVCHTWEPPY-QGWMMVDCTCLGFGSGRITCTSRNR
 6   CNDQDTRTSYRIGDTWSKKDNRGN-LLQCICTGNGRGEWKCER
 7   HTSVQTTSSGSGPFTDVRAAVYQPQPHPQPPPYGH
 8   CVIDS-GVVYSVGMQWLK--TQGNKQMLCTCLGNG---VSCQE
 9   TAVTQTWGGNSNGEPCVLPFTYNGRTFYSCTTEGRQDGHLWCSTTSNYEQDQKYSFCTDH
10   TVLVQTQGGNSNGALCHFPFLYNNHNYTDCTSEGRRDNHKWCGTTQNYDADQKFGFCPMAAHEEI
11   CTTNE-SVMYRIGDQMDKQHD-HGHHMRCTCVGNGRGEWTCYAYSQLRDQ
12   CIVD--DITYNVNDTFHKRHE-EGHMLNCTCFGQGRGRWKCDPVDQ
13   CQDSETGTFYQIGDSWEK--YVHGVRYQCYCYGRGIGEWHCQPLQTYPSS
14   SGPVEVFITETPSQPNSHP-IQWNAPQPSHISKYILRWRPKNSVGRWKEATIPGHLNSY-TIIKGLKPGVVYEGQLISIQQ----YGHQEVTRFDFTTI
15   STSTPVTSNTVTGETTPF
16   SPLVATSESVTIIASSFV-VSWVSASDT-VSGFKVEYELSEEGDEPQYLDLPSTATSV-NIPDLLPGRKYIVNVYQISE----DGEQSLILSTROTI
17   APDAFPDPTVDQVDQTSIV-VRWSRPIQAP-ITGYRIVYSPSVEGSSTELN-LPETANSV-TLSDLQPGVQYNITIYAVEE----NQE-STPVVIQQETTGTPRSD
18   TVPSPRDLQFVEYTDVKVT-IMWTPPESA-VTGYRVIPVNLPGEHGQRLPISRNTFA-EVTGLSPGVTYYFKVFAVSH----GRE-SKPLTAQQTI
19   KLDAPTNLQFVNETDSTVI-VRWTPPRAQ-ITGYRLTVGLTRRGQPRQYNVGPSVSKY--PLRMLQPASEYTVSLVAIKG----NQE-SPKATGVFTTL
20   QPQSSIPPYNTEVTETTIV-ITWTPAPRIGFKLGVRPSQGGEAPREVTSDSG-----SI-VVSGLTPGVEYVYTIQVLRD----GQERDAPIVNKVVT
21   PLSPPTNLHLEANPDTGVLTVSWERSTTPDITGYRITTTPTNGQQGNSLEEVVHADQSSCTFDNLSPGLEYNVSVYTVKD----DKE-SVPISDTIIP
22   AVPPPTDLRFTNIGPDTNR-VTWAPPPSIDLTNFLVRYSPVKNEEDVAELSISPSDNAV-VLTNLLPGTEY-IVSVSSVYE----QHE-STPLPGROKT
23   GLDSPTGIDFSDITANSFT-VHWIAPRAT-ITGYRIRHHPEHFSGRPREDRVPHSRNSI-ILTNLTPGTEYVVSIVALNG----REE-SPLLIGQQST
24   VSDVPRDLEVVAATPTSLL-ISWDAPAVT-VRYYRITYGETGGNSPVQEFTVPGSKSTA-TISGLKPGVDYTITVYAVTGRGDSPAS-SKPISINYRT
25   EIDKPSQMQVTDVQDNSIS-VKWLPSSSP-VTGYRVTTTPKNGPGPTKTKTAGPDQTEM-TIEGLQPTVEYVVSVYAQNP----SGE-SQPLVQTAVT
26   NIIQRPKGLAFTDVDVDSIK-IAWESPQGQ-VSRYRVTYSSPEDGIHELFPAPDGEEDTA-ELQGLRPGSEYTVSVVALHD----DME-SQPLIGTQST
27   ALPAPTDLKFTQVTPTSLS-AQWTPPNVQ-LTGYRVRVTPKEKTGPMKEINLAPDSSSV-VVSGLMVATKYEVSVYALKD----TLT-SRPAQGVVTTLE
28   NVSPPRRARVTDATETTIT-ISWRTKTET-ITGFQVDAVPANGQTPIQRT-IKPDVRSY-TITGLQPGTDYKIYLYTLND----NAR-SISPVVIDAST
29   ALDAPSNLRFLATTPNSLL-VSMQPPRAR-ITGYIIKYEKPGSPPREVVPRPRPGVTEA-D-TGLEPGTEYTIYVIALKN----NQK-SEPLIGRKKT
30   CSDELPQLVTLPHPNLFGPEILDVPSTVQKTPFVTHPGYCTGNGIQLPGTSG-QQPSVGQQMIFEEMGFRRTTPPTTATPIRHRPRPYPPNV
31   GQEALSQTT-ISWAPFQDT--SEYIISCHPVGTDEEPLQFRVPGTSTSA-TLTGLTRGATYNIVEALKD----QQR-HKA
32   VREEVVFVGNSVNEGLNQPTDDS
33   CFDPYTVSHVAVGDEWERMSESGFKLLCQCLGFGSGHFRCDSSRW
34   CHNGV--NYKIGEKWDRQGENGQMMSCTCLGNGKGEFKCDPHEAT
35   GVDQGKT--VHVGEUWQK--EYLGAICSCTCFGGQRGWR-CDNCRRPG
36   GEPSPEGTTGQSYNQYSQRYHQRTNTNVNCPIECFNPLDVQADREDSRE
```

Section labels (left): I (lines 2–6), II (lines 9–10), I (lines 11–13), III (line 14), III (line 23), ED (lines 24–29), III (line 30), III (line 31), I (lines 33–35)

Binding sites (right):
FIBRIN HEPARIN S AUREUS
COLLAGEN
DNA
CELLS
HEPARIN
FIBRIN

# Fig.3a.

FIRST SEQ. NO.=

S K R Q A Q Q M V Q P Q S P V A V S Q S K P G C Y D N G K H Y Q I N Q Q W E R
GAAGAGCAAGAGGCAGGCTCAGCAAATGGTTCAGCCCCAGTCCCCGGTGGCTGTCAGTCAAAGCAAGCCCGGTTGTTATGACAATGGAAAACACTATCAGATAAATCAACAGTGGGAGCG

T Y L G N V L V C T C Y G G S R G F N C E S K P E A E E T C F D K Y T G N T Y R
GACCTACCTAGGTAATGTGTTGGTTTGTACTTGTTATGGAGGAAGCCGAGGTTTTAACTGCGAAAGTAAACCTGAAGCTGAAGAGACTTGCTTTGACAAGTACACTGGGAACACTTACCG

V G D T Y E R P K D S M I W D C T C I G A G R G R I S C T I A N R C H E G G Q S
AGTGGGTGACACTTATGAGCGTCCTAAAGACTCCATGATCTGGGACTGTACCTGCATCGGGGCTGGGCGAGGGAGAATAAGCTGTACCATCGCAAACCGCTGCCATGAAGGGGGTCAGTC

Y K I G D T W R R P H E T G G Y M L E C V C L G N G K G E W T C K P I A E K C F
CTACAAGATTGGTGACACCTGGAGGAGACCACATGAGACTGGTGGTTACATGTTAGAGTGTGTGTGTCTTGGTAATGGAAAAGGAGAATGGACCTGCAAGCCCATAGCTGAGAAGTGTTT

D H A A G T S Y V V G E T W E K P Y Q G W M M V D C T C L G E G S G R I T C T S
TGATCATGCTGCTGGGACTCCCTATGTGGTCGGAGAAACGTGGGAGAAGCCCTACCAAGGCTGGATGATGGTAGATTGTACTTGCCTGGGAGAAGGCAGCGGACGCATCACTTGCACTTC

R N R C N D Q D T R T S Y R I G D T W S K K D N R G N L L Q C I C T G N G R G E
TAGAAATAGATGCAACGATCAGGACACAAGGACATCCTATAGAATTTGAGACACCTGGAGCAAGAAGGATAATCGAGGAAACCTGCTCCAGTGCATCTGCACAGGCAACGGCCGAGGAGA

W K C E R H T S V Q T T S S G S G P F T D V R A A V Y Q P Q P H P Q P P P Y G H
GTGGAAGTGTGAGAGGCACACCTCTGTGCAGACCACATCGAGCGGATCTGGCCCCTTCACCGATGTTCGTGCAGCTGTTTACCAACCGCAGCCTCACCCCCAGCCTCCTCCCTATGGCCA

C V T D S G V V Y S V G M Q W L K T Q G N K Q M L C T C L G N G V S C Q E T A V
CTGTGTCACAGACAGTGGTGTGGTCTACTCTGTGGGGATGCAGTGGTTGAAGACACAAGGAAATAAGCAAATGCTTTGCACGTGCCTGGGCAACGGAGTCAGCTGCCAAGAGACAGCTGT

## Fig.3b.

```
    T  Q  T  Y  G  G  N  L  N  G  E  P  C  V  L  P  F  T  Y  N  G  R  T  F  Y  S  C  T  T  E  G  R  Q  D  G  H  L  W  C  S
AACCCAGACTTACGGTGGCAACTTAAATGGAGAGCCATGTGTCTTACCATTCACCTACAATGGCAGGACGTTCTACTCCTGCACCACGGAAGGGCGACAGGACGGACATCTTTGGTGCAG
     970        980        990       1000       1010       1020       1030       1040       1050       1060       1070       1080
                                                                 10
    T  T  S  N  Y  E  Q  D  Q  K  Y  S  F  C  T  D  H  T  V  L  V  Q  T  Q  G  G  N  S  N  G  A  L  C  H  F  P  F  L  Y  N
CACAACTTCGAATTATGAGCAGGACCAGAAATACTCTTTCTGCACAGACCACACTGTTTTGGTTCAGACTCAAGGAGGAAATTCCAATGGTGCCTTGTGCCACTTCCCCTTCCTATACAA
    1090       1100       1110       1120       1130       1140       1150       1160       1170       1180       1190       1200

    N  H  N  Y  T  D  C  T  S  E  G  R  R  D  N  M  K  W  C  G  T  T  Q  N  Y  D  A  D  Q  K  F  G  F  C  P  M  A  A  H  E
CAACCACAATTACACTGATTGCACTTCTGAGGGCAGAAGAGACAACATGAAGTGGTGTGGGACCACACAGAACTATGATGCCGACCAGAAGTTTGGGTTCTGCCCCATGGCTGCCCACGA
    1210       1220       1230       1240       1250       1260       1270       1280       1290       1300       1310       1320
        11
    E  I  C  T  T  N  E  G  V  M  Y  R  I  G  D  Q  W  D  K  Q  H  D  M  G  H  M  M  R  C  T  C  V  G  N  R  G  E  W  T
GGAAATCTGCACAACCAATGAAGGGGTCATGTACCGCATTGGAGATCAGTGGGATAAGCAGCATGACATGGGTCACATGATGAGGTGCACGTGTGTTGGGAATGGTCGTGGGGAATGGAC
    1330       1340       1350       1360       1370       1380       1390       1400       1410       1420       1430       1440
                                    12
    C  I  A  Y  S  Q  L  R  D  Q  C  I  V  D  D  I  T  Y  N  V  N  D  T  F  H  K  R  H  E  E  G  H  M  L  N  C  T  C  F  G
ATGCATTGCCTACTCGCAACTTCGAGATCAGTGCATTGTTGATGACATCACTTACAATGTGAACGACACATTCCACAAGCGTCATGAAGAGGGGCACATGCTGAACTGTACATGCTTCGG
    1450       1460       1470       1480       1490       1500       1510       1520       1530       1540       1550       1560
                                13
    Q  G  R  G  R  W  K  C  D  P  V  D  Q  C  Q  D  S  E  T  G  T  F  Y  Q  I  G  D  S  W  E  K  Y  V  H  G  V  R  Y  Q  C
TCAGGGTCGGGGCAGGTGGAAGTGTGATCCCGTCGACCAATGCCAGGATTCAGAGACTGGGACGTTTTATCAAATTGGAGATTCATGGGAGAAGTATGTGCATGGTGTCAGATACCAGTG
    1570       1580       1590       1600       1610       1620       1630       1640       1650       1660       1670       1680
                                        14
    Y  C  Y  G  R  G  I  G  E  W  H  C  Q  P  L  Q  T  Y  P  S  S  S  G  P  V  E  V  F  I  T  E  T  P  S  Q  P  N  S  H  P
CTACTGCTATGGCCGTGGCATTGGGGAGTGGCATTGCCAACCTTTACAGACCTATCCAAGCTCAAGTGGTCCTGTCGAAGTATTTATCACTGAGACTCCGAGTCAGCCCAACTCCCACCC
    1690       1700       1710       1720       1730       1740       1750       1760       1770       1780       1790       1800

    I  Q  W  N  A  P  Q  P  S  H  I  S  K  Y  I  L  R  W  R  P  K  N  S  V  G  R  W  K  E  A  T  I  P  G  H  L  N  S  Y  T
CATCCAGTGGAATGCACCACAGCCATCTCACATTTCCAAGTACATTCTCAGGTGGAGACCTAAAAATTCTGTAGGCCGTTGGAAGGAAGCTACCATACCAGGCCACTTAAACTCCTACAC
    1810       1820       1830       1840       1850       1860       1870       1880       1890       1900       1910       2000
```

EP 0 207 751 B1

# Fig.3c.

```
 I  K  G  L  K  P  G  V  V  Y  E  G  Q  L  I  S  I  Q  Q  Y  G  H  Q  E  V  T  R  F  D  F  T  T  T  S  T  S  T  P  V  T
CATCAAAGGCCTGAAGCCTGGTGTGGTATACGAGGGCCAGCTCATCAGCATCCAGCAGTACGGCCACCAAGAAGTGACTCGCTTTGACTTCACCACCACCAGCACCAGCACACCTGTGAC
     1930      1940      1950      1960      1970      1980      1990      2000      2010      2020      2030      2040

 S  N  T  V  T  G  E  T  T  P  F  S  P  L  V  A  T  S  E  S  V  T  E  I  T  A  S  S  F  V  V  S  W  V  S  A  S  D  T  V
CAGCAACACCGTGACAGGAGAGACGACTCCCTTTTCTCCTCTTGTGGCCACTTCTGAATCTGTGACCGAAATCACAGCCAGTAGCTTTGTGGTCTCCTGGGTCTCAGCTTCCGACACCGT
     2050      2060      2070      2080      2090      2100      2110      2120      2130      2140      2150      2160

 S  G  F  R  V  E  Y  E  L  S  E  E  G  D  E  P  Q  Y  L  D  L  P  S  T  A  T  S  V  N  I  P  D  L  L  P  G  R  K  Y  I
GTCGGGATTCCGGGTGGAATATGAGCTGAGTGAGGAGGGAGATGAGCCACAGTACCTGGATCTTCCAAGCACAGCCACTTCTGTGAACATCCCTGACCTGCTTCCTGGCCGAAAATACAT
     2170      2180      2190      2200      2210      2220      2230      2240      2250      2260      2270      2280

 V  N  V  Y  Q  I  S  E  D  G  E  Q  S  L  I  L  S  T  S  Q  T  T  A  P  D  A  P  P  D  P  T  V  D  Q  V  D  D  T  S  I
TGTAAATGTCTATCAGATATCTGAGGATGGGGAGCAGAGTTTGATCCTGTCTACTTCACAAACAACAGCGCCTGATGCCCCTCCTGACCCGACTGTGGACCAAGTTGATGACACCTCAAT
     2290      2300      2310      2320      2330      2340      2350      2360      2370      2380      2390      2400

 V  V  R  W  S  R  P  Q  A  P  I  T  G  Y  R  I  V  Y  S  P  S  V  E  G  S  S  T  E  L  N  L  P  E  T  A  N  S  V  T  L
TGTTGTTCGCTGGAGCAGACCCCAGGCTCCCATCACAGGGTACAGAATAGTCTATTCGCCATCAGTAGAAGGTAGCAGCACAGAACTCAACCTTCCTGAAACTGCAAACTCCGTCACCCT
     2410      2420      2430      2440      2450      2460      2470      2480      2490      2500      2510      2520

 S  D  L  Q  P  G  V  Q  Y  N  I  T  I  Y  A  V  E  E  N  Q  E  S  T  P  V  V  I  Q  Q  E  T  T  G  T  P  R  S  D  T  V
CAGTGACTTGCAACCTGGTGTTCAGTATAACATCACTATCTATGCTGTGGAAGAAAATCAAGAAGTACACCTGTTGTCATTCAACAAGAAACCACTGGCACCCCACGCTCAGATACAGT
     2530      2540      2550      2560      2570      2580      2590      2600      2610      2620      2630      2640

 P  S  P  R  D  L  Q  F  V  E  V  T  D  V  K  V  T  I  M  W  T  P  P  E  S  A  V  T  G  Y  R  V  D  V  I  P  V  N  L  P
GCCCTCTCCCAGGGACCTGCAGTTTGTGGAAGTGACAGACGTGAAGGTCACCATCATGTGGACACCGCCTGAGAGTGCAGTGACCGGCTACCGTGTGGATGTGATCCCCGTCAACCTGCC
     2650      2660      2670      2680      2690      2700      2710      2720      2730      2740      2750      2760

 G  E  H  G  Q  R  L  P  I  S  R  N  T  F  A  E  V  T  G  L  S  P  G  V  T  Y  Y  F  K  V  F  A  V  S  H  G  R  E  S  K
TGGCGAGCACGGGCAGAGGCTGCCCATCAGCAGGAACACCTTTGCAGAAGTCACCGGGCTGTCCCCTGGGGTCACCTATTACTTCAAAGTCTTTGCAGTGAGCCATGGGAGGGAGAGCAA
     2770      2780      2790      2800      2810      2820      2830      2840      2850      2860      2870      2880
```

EP 0 207 751 B1

# Fig. 3d.

```
                     19
     P  L  T  A  Q  Q  T  T  K  L  D  A  P  T  N  L  Q  F  V  N  E  T  D  S  T  V  L  V  R  W  T  P  P  R  A  Q  I  T  G  Y
GCCTCTGACTGCTCAACAGACAACCAAACTGGATGCTCCCACTAACCTCCAGTTTGTCAATGAAACTGATTCTACTGTCCTGGTGAGATGGACTCCACCTCGGGCCCAGATAACAGGATA
     2890      2900      2910      2920      2930      2940      2950      2960      2970      2980      2990      3000

     R  L  T  V  G  L  T  R  R  G  Q  P  R  Q  Y  N  V  G  P  S  V  S  K  Y  P  L  R  N  L  Q  P  A  S  E  Y  T  V  S  L  V
CCGACTGACCGTGGGCCTTACCCGAAGAGGCCAGCCCAGGCAGTACAATGTGGGTCCCTCTGTCTTCAAGTACCCCCTGAGGAATCTGCAGCCTGCATCTGAGTACACCGTATCCCTCGT
     3010      3020      3030      3040      3050      3060      3070      3080      3090      3100      3110      3120

                                                    20
     A  I  K  G  N  Q  E  S  P  K  A  T  G  V  F  T  T  L  Q  P  G  S  S  I  P  P  Y  N  T  E  V  T  E  T  T  I  V  I  T  W
GGCCATAAAGGGCAACCAAGAGAGCCCCAAAGCCACTGGAGTCTTTACCACACTGCAGCCTGGGAGCTCTATTCCACCTTACAACACCGAGGTGACTGAGACCACCATCGTGATCACATG
     3130      3140      3150      3160      3170      3180      3190      3200      3210      3220      3230      3240

     T  P  A  P  R  I  G  F  K  L  G  V  R  P  S  Q  G  G  E  A  P  R  E  V  T  S  D  S  G  S  I  V  V  S  G  L  T  P  G  V
GACGCCTGCTCCAAGAATTGGTTTTAAGCTGGGTGTACGACCAAGCCAGGGAGGAGAGGCACCACGAGAAGTGACTTCAGACTCAGGAAGCATCGTTGTGTCCGGCTTGACTCCAGGAGT
     3250      3260      3270      3280      3290      3300      3310      3320      3330      3340      3350      3360

                                                         21
     E  Y  V  Y  Y  T  I  Q  V  L  R  D  G  Q  E  R  D  A  P  I  V  N  K  V  V  T  P  L  S  P  P  T  N  L  H  L  E  A  N  P  D
AGAATACGTCTACACCATCCAAGTCCTGAGAGATGGACAGGAAAGAGATGCGCCAATTGTAAACAAAGTGGTGACACCATTGTCTCCACCAACAAACTTGCATCTGGAGGCAAACCCTGA
     3370      3380      3390      3400      3410      3420      3430      3440      3450      3460      3470      3480

     T  G  V  L  T  V  S  W  E  R  S  T  T  P  D  I  T  G  Y  R  I  T  T  T  P  T  N  G  Q  Q  G  N  S  L  E  E  V  V  H  A
CACTGGAGTGCTCACAGTCTCCTGGGAGAGGAGCACCACCCCAGACATTACTGGTTATAGAATTACCACAACCCCTACAAACGGCCAGCAGGGAAATTCTTTGGAAGAAGTGGTCCATGC
     3490      3500      3510      3520      3530      3540      3550      3560      3570      3580      3590      3600

                                                                                                            22
     D  Q  S  S  C  T  F  D  N  L  S  P  G  L  E  Y  N  V  S  V  Y  T  V  K  D  D  K  E  S  V  P  I  S  D  T  I  I  P  A  V
TGATCAGAGCTCCTGCACTTTTGATAACCTGAGTCCCGGCCTGGAGTACAATGTCAGTGTTTACACTGTCAAGGATGACAAGGAAAGTGTCCCTATCTCTGATACCATCATCCCAGCTGT
     3610      3620      3630      3640      3650      3660      3670      3680      3690      3700      3710      3720

     P  P  P  T  D  L  R  F  T  N  I  G  P  D  T  M  R  V  T  W  A  P  P  P  S  I  D  L  T  N  F  L  V  R  Y  S  P  V  K  N
TCCTCCTCCCACTGACCTGCGATTCACCAACATTGGTCCAGACACCATGCGTGTCACCTGGGCTCCACCCCCCATCCATTGATTTAACCAACTTCCTGGTGCGTTACTCACCTGTGAAAAA
     3730      3740      3750      3760      3770      3780      3790      3800      3810      3820      3830      3840
```

EP 0 207 751 B1

## Fig.3e.

```
    E  E  D  V  A  E  L  S  I  S  P  S  D  N  A  V  V  L  T  N  L  L  P  G  T  E  Y  V  V  S  V  S  S  V  Y  E  Q  H  E  S
TGAGGAAGATGTTGCAGAGTTGTCAATTTCTCCTTCAGACAATGCAGTGGTCTTAACAAATCTCCTGCCTGGTACAGAATATGTAGTGAGTGTCTCCAGTGTCTACGAACAACATGAGAG
      3850      3860      3870      3880      3890      3900      3910      3920      3930      3940      3950      3960

                    23
    T  P  L  R  G  R  Q  K  T  G  L  D  S  P  T  G  I  D  F  S  D  I  T  A  N  S  F  T  V  H  W  I  A  P  R  A  T  I  T  G
CACACCTCTTAGAGGAAGACAGAAAACAGGTCTTGATTCCCCAACTGGCATTGACTTTTCTGATATTACTGCCAACTCTTTTACTGTGCACTGGATTGCTCCTCGAGCCACCATCACTGG
      3970      3980      3990      4000      4010      4020      4030      4040      4050      4060      4070      4080


    Y  R  I  R  H  H  P  E  H  F  S  G  R  P  R  E  D  R  V  P  H  S  R  N  S  I  T  L  T  N  L  T  P  G  T  E  Y  V  V  S
CTACAGGATCCGCCATCATCCCGAGCACTTCAGTGGGAGACCTCGAGAAGATCGGGTGCCCCACTCTCGGAATTCCATCACCCTCACCAACCTCACTCCAGGCACAGAGTATGTGGTCAG
      4090      4100      4110      4120      4130      4140      4150      4160      4170      4180      4190      4200

                                              24
    I  V  A  L  N  G  R  E  E  S  P  L  L  I  G  Q  Q  S  T  V  S  D  V  P  R  D  L  E  V  V  A  A  T  P  T  S  L  L  I  S
CATCGTTGCTCTTAATGGCAGAGAGGAAAGTCCCTTATTGATTGGCCAACAATCAACAGTTTCTGATGTTCCGAGGGACCTGGAAGTTGTTGCTGCGACCCCCACCAGCCTACTGATCAG
      4210      4220      4230      4240      4250      4260      4270      4280      4290      4300      4310      4320


    W  D  A  P  A  V  T  V  R  Y  Y  R  I  T  Y  G  E  T  G  G  N  S  P  V  Q  E  F  T  V  P  G  S  K  S  T  A  T  I  S  G
CTGGGATGCTCCTGCTGTCACAGTGAGATATTACAGGATCACTTACGGAGAAACAGGAGGAAATAGCCCTGTCCAGGAGTTCACTGTGCCTGGGAGCAAGTCTACAGCTACCATCAGCGG
      4330      4340      4350      4360      4370      4380      4390      4400      4410      4420      4430      4440

                                                                                          25
    L  K  P  G  V  D  Y  T  I  T  V  Y  A  V  T  G  R  G  D  S  P  A  S  S  K  P  I  S  I  N  Y  R  T  E  I  D  K  P  S  Q
CCTTAAACCTGGAGTTGATTATACCATCACTGTGTATGCTGTCACTGGCCGTGGAGACAGCCCCGCAAGCAGCAAGCCAATTTCCATTAATTACCGAACAGAAATTGACAAACCATCCCA
      4450      4460      4470      4480      4490      4500      4510      4520      4530      4540      4550      4560


    M  Q  V  T  D  V  Q  D  N  S  I  S  V  K  W  L  P  S  S  S  P  V  T  G  Y  R  V  T  T  T  P  K  N  G  P  G  P  T  K  T
GATGCAAGTGACCGATGTTCAGGACAACAGCATTAGTGTCAAGTGGCTGCCTTCAAGTTCCCCTGTTACTGGTTACAGAGTAACCACCACTCCCAAAAATGGACCAGGACCAACAAAAAC
      4570      4580      4590      4600      4610      4620      4630      4640      4650      4660      4670      4680


    K  T  A  G  P  D  Q  T  E  M  T  I  E  G  L  Q  P  T  V  E  Y  V  V  S  V  Y  A  Q  N  P  S  G  E  S  Q  P  L  V  Q  T
TAAAACTGCAGGTCCAGATCAAACAGAAATGACTATTGAAGGCTTGCAGCCCACAGTGGAGTATGTGGTTAGTGTCTATGCTCAGAATCCAAGCGGAGAGAGTCAGCCTCTGGTTCAGAC
      4690      4700      4710      4720      4730      4740      4750      4760      4770      4780      4790      4800
```

EP 0 207 751 B1

## Fig. 3f.

```
          26
A  V  T  N  I  D  R  P  K  G  L  A  F  T  D  V  D  V  D  S  I  K  I  A  W  E  S  P  Q  G  Q  V  S  R  Y  R  V  T  Y  S
TGCAGTAACCAACATTGATCGCCCTAAAGGACTGGCATTCACTGATGTGGATGTCGATTCCATCAAAATTGCTTGGGAAAGCCCACAGGGGCAAGTTTCCAGGTACAGGGTGACCTACTC
   4810      4820      4830      4840      4850      4860      4870      4880      4890      4900      4910      4920


S  P  E  D  G  I  H  E  L  F  P  A  P  D  G  E  E  D  T  A  E  L  Q  G  L  R  P  G  S  E  Y  T  V  S  V  V  A  L  H  D
GAGCCCTGAGGATGGAATCCATGAGCTATTCCCTGCACCTGATGGTGAAGAAGACACTGCAGAGCTGCAAGGCCTCAGACCGGGTTCTGAGTACACAGTCAGTGTGGTTGCCTTGCACGA
   4930      4940      4950      4960      4970      4980      4990      5000      5010      5020      5030      5040


            27
D  M  E  S  Q  P  L  I  G  T  Q  S  T  A  I  P  A  P  T  D  L  K  F  T  Q  V  T  P  T  S  L  S  A  Q  W  T  P  P  N  V
TGATATGGAGAGCCAGCCCCTGATTGGAACCCAGTCCACAGCTATTCCTGCACCAACTGACCTGAAGTTCACTCAGGTCACACCCACAAGCCTGAGCGCCCAGTGGACACCACCCAATGT
   5050      5060      5070      5080      5090      5100      5110      5120      5130      5140      5150      5160


Q  L  T  G  Y  R  V  R  V  T  P  K  E  K  T  G  P  M  K  E  I  N  L  A  P  D  S  S  S  V  V  V  S  G  L  M  V  A  T  K
TCAGCTCACTGGATATCGAGTGCGGGTGACCCCCAAGGAGAAGACCGGACCAATGAAAGAAATCAACCTTGCTCCTGACAGCTCATCCGTGGTTGTATCAGGACTTATGGTGGCCACCAA
   5170      5180      5190      5200      5210      5220      5230      5240      5250      5260      5270      5280


                                                       28
Y  E  V  S  V  Y  A  L  K  D  T  L  T  S  R  P  A  Q  G  V  V  T  T  L  E  N  V  S  P  P  R  R  A  R  V  T  D  A  T  E
ATATGAAGTGAGTGTCTATGCTCTTAAGGACACTTTGACAAGCAGACCAGCTCAGGGTGTTGTCACCACTCTGGAGAATGTCAGCCCACCAAGAAGGGCTCGTGTGACAGATGCTACTGA
   5290      5300      5310      5320      5330      5340      5350      5360      5370      5380      5390      5400


T  T  I  T  I  S  W  R  T  K  T  E  T  I  T  G  F  Q  V  D  A  V  P  A  N  G  Q  T  P  I  Q  R  T  I  K  P  D  V  R  S
GACCACCATCACCATTAGCTGGAGAACCAAGACTGAGACGATCACTGGCTTCCAAGTTGATGCCGTTCCAGCCAATGGCCAGACTCCAATCCAGAGAACCATCAAGCCAGATGTCAGAAG
   5410      5420      5430      5440      5450      5460      5470      5480      5490      5500      5510      5520


                                                                      29
Y  T  I  T  G  L  Q  P  G  T  D  Y  K  I  Y  L  Y  T  L  N  D  N  A  R  S  S  P  V  V  I  D  A  S  T  A  I  D  A  P  S
CTACACCATCACAGGTTTACAACCAGGCACTGACTACAAGATCTACCTGTACACCTTGAATGACAATGCTCGGAGCTCCCCTGTGGTCATCGACGCCTCCACTGCCATTGATGCACCATC
   5530      5540      5550      5560      5570      5580      5590      5600      5610      5620      5630      5640


N  L  R  F  L  A  T  T  P  N  S  L  L  V  S  W  Q  P  P  R  A  R  I  T  G  Y  I  I  K  Y  E  K  P  G  S  P  P  R  E  V
CAACCTGCGTTTCCTGGCCACCACACCCAATTCCTTGCTGGTATCATGGCAGCCGCCACGTGCCAGGATTACCGGCTACATCATCAAGTATGAGAAGCCTGGGTCTCCTCCCAGAGAAGT
   5650      5660      5670      5680      5690      5700      5710      5720      5730      5740      5750      5760
```

EP 0 207 751 B1

# Fig.3g.

```
    V  P  R  P  R  P  G  V  T  E  A  T  I  T  G  L  E  P  G  T  E  Y  T  I  Y  V  I  A  L  K  N  N  Q  K  S  E  P  L  I  G
GGTCCCTCGGCCCCGCCCTGGTGTCACAGAGGCTACTATTACTGGCCTGGAACCGGGAACCGAATATACAATTTATGTCATTGCCCTGAAGAATAATCAGAAGAGCGAGCCCCTGATTGG
   5770      5780      5790      5800      5810      5820      5830      5840      5850      5860      5870      5880

          30
    R  K  K  T  D  E  L  P  Q  L  V  T  L  P  H  P  N  L  H  G  P  E  I  L  D  V  P  S  T  V  Q  K  T  P  F  V  T  H  P  G
AAGGAAAAAGACAGACGAGCTTCCCCAACTGGTAACCCTTCCACACCCCAATCTTCATGGACCAGAGATCTTGGATGTTCCTTCCACAGTTCAAAAGACCCCTTTCGTCACCCACCCTGG
   5890      5900      5910      5920      5930      5940      5950      5960      5970      5980      5990      6000

    Y  D  T  G  N  G  I  Q  L  P  G  T  S  G  Q  Q  P  S  V  G  Q  Q  M  I  F  E  E  H  G  F  R  R  T  T  P  P  T  T  A  T
GTATGACACTGGAAATGGTATTCAGCTTCCTGGCACTTCTGGTCAGCAACCCAGTGTTGGGCAACAAATGATCTTTGAGGAACATGGTTTTAGGCGGACCACACCGCCCACAACGGCCAC
   6010      6020      6030      6040      6050      6060      6070      6080      6090      6100      6110      6120

                                              31
    P  I  R  H  R  P  R  P  Y  P  P  N  V  G  Q  E  A  L  S  Q  T  T  I  S  W  A  P  F  Q  D  T  S  E  Y  I  I  S  C  H  P
CCCCATAAGGCATAGGCCAAGACCATACCCGCCGAATGTAGGACAAGAAGCTCTCTCTCAGACAACCATCTCATGGGCCCCATTCCAGGACACTTCTGAGTACATCATTTCATGTCATCC
   6130      6140      6150      6160      6170      6180      6190      6200      6210      6220      6230      6240

    V  G  T  D  E  E  P  L  Q  F  R  V  P  G  T  S  T  S  A  T  L  T  G  L  T  R  G  A  T  Y  N  I  I  V  E  A  L  K  D  Q
TGTTGGCACTGATGAAGAACCCTTACAGTTCAGGGTTCCTGGAACTTCTACCAGTGCCACTCTGACAGGCCTCACCAGAGGTGCCACCTACAACATCATAGTGGAGGCACTGAAAGACCA
   6250      6260      6270      6280      6290      6300      6310      6320      6330      6340      6350      6360

          32                                                                    33
    Q  R  H  K  V  R  E  E  V  V  T  V  G  N  S  V  N  E  G  L  N  Q  P  T  D  D  S  C  F  D  P  Y  T  V  S  H  Y  A  V  G
GCAGAGGCATAAGGTTCGGGAAGAGGTTGTTACCGTGGGCAACTCTGTCAACGAAGGCTTGAACCAACCTACGGATGACTCGTGCTTTGACCCCTACACAGTTTCCCATTATGCCGTTGG
   6370      6380      6390      6400      6410      6420      6430      6440      6450      6460      6470      6480

                                                                              34
    D  E  W  E  R  M  S  E  S  G  F  K  L  L  C  Q  C  L  G  F  G  S  G  H  F  R  C  D  S  S  R  W  C  H  D  N  G  V  N  Y
AGATGAGTGGGAACGAATGTCTGAATCAGGCTTTAAACTGTTGTGCCAGTGCTTAGGCTTTGGAAGTGGTCATTTCAGATGTGATTCATCTAGATGGTGCCATGACAATGGTGTGAACTA
   6490      6500      6510      6520      6530      6540      6550      6560      6570      6580      6590      6600

                                                                                      35
    K  I  G  E  K  W  D  R  Q  G  E  N  G  Q  M  M  S  C  T  C  L  G  N  G  K  G  E  F  K  C  D  P  H  E  A  T  C  Y  D  D
CAAGATTGGAGAGAAGTGGGACCGTCAGGGAGAAAATGGCCAGATGATGAGCTGCACATGTCTTGGGAACGGAAAAGGAGAATTCAAGTGTGACCCTCATGAGGCAACGTGTTACGATGA
   6610      6620      6630      6640      6650      6660      6670      6680      6690      6700      6710      6720
```

EP 0 207 751 B1

## Fig. 3h.

EP 0 207 751 B1

```
      G  K  T  Y  H  V  G  E  Q  W  Q  K  E  Y  L  G  A  I  C  S  C  T  C  F  G  G  Q  R  G  W  R  C  D  N  C  R  R  P  G 36 G
TGGGAAGACATACCACGTAGGAGAACAGTGGCAGAAGGAATATCTCGGTGCCATTTGCTCCTGCACATGCTTTGGAGGCCAGCGGGGCTGGCGCTGTGACAACTGCCGCAGACCTGGGGG
      6730     6740     6750     6760     6770     6780     6790     6800     6810     6820     6830     6840


      E  P  S  P  E  G  T  T  G  Q  S  Y  N  Q  Y  S  Q  R  Y  H  Q  R  T  N  T  N  V  N  C  P  I  E  C  F  M  P  L  D  V  Q
TGAACCCAGTCCCGAAGGCACTACTGGCCAGTCCTACAACCAGTATTCTCAGAGATACCATCAGAGAACAAACACTAATGTTAATTGCCCAATTGAGTGCTTCATGCCTTTAGATGTACA
      6850     6860     6870     6880     6890     6900     6910     6920     6930     6940     6950     6960


      A  D  R  E  D  S  R  E
GGCTGACAGAGAAGATTCCCGAGAGTAAATCATCTTTCCAATCCAGAGGAACAAGCATGTCTCTCTGCCAAGATCCATCTAAACTGGAGTGATGTTAGCAGACCCAGCTTAGAGTTCTTC
      6970     6980     6990     7000     7010     7020     7030     7040     7050     7060     7070     7080


TTTCTTTCTTAAGCCCTTTGCTCTGGAGGAAGTTCTCCAGCTTCAGCTCAACTCACAGCTTCTCCAAGCATCACCCTGGGAGTTTCCTGAGGGTTTTCTCATAAATGAGGGCTGCACATT
      7090     7100     7110     7120     7130     7140     7150     7160     7170     7180     7190     7200


GCCTGTTCTGCTTCGAAGTATTCAATACCGCTCAGTATTTTAAATGAAGTGATTCTAAGATTTGGTTTGGGATCAATAGGAAAGCATATGCAGCCAACCAAGATGCAAATGTTTTGAAAT
      7210     7220     7230     7240     7250     7260     7270     7280     7290     7300     7310     7320


GATATGACCAAAATTTTAAGTAGGAAAGTCACCCAAACACTTCTGCTTTCACTTAAGTGTCTGGCCCGCAATACTGTAGGAACAAGCATGATCTTGTTACTGTGATATTTTAAATATCCA
      7330     7340     7350     7360     7370     7380     7390     7400     7410     7420     7430     7440


CAGTACTCACTTTTTCCAAATGATCCTAGTAATTGCCTAGAAATATCTTTCTCTTACCTGTTATTTATCAATTTTTCCCAGTATTTTTATACGGAAAAAATTGTATTGAAAACACTTAGT
      7450     7460     7470     7480     7490     7500     7510     7520     7530     7540     7550     7560


ATGCAGTTGATAAGAGGAATTTGGTATAATTATGGTGGGTGATTATTTTTTATACTGTATGTGCCAAAGCTTTACTACTGTGGAAAGACAACTGTTTAATAAAAGATTTACATTCCACA
      7570     7580     7590     7600     7610     7620     7630     7640     7650     7660     7670     7680


AAAAAAAAAAAAAAAAAAAAAAAA
      7690     7700      5       15      25      35      45      55      65      75      85      95
```

Fig. 4.

Fig.5.

COLLAGEN BINDING

PROTEIN